# FASCICULE DE BREVET EUROPEEN

(11) **EP 0 789 699 B1**
(45) Date de publication et mention de la délivrance du brevet: **17.03.1999**
(21) Numéro de dépôt: 95936624.6
(22) Date de dépôt: 30.10.1995
(51) Int. Cl.: C07D 487/20, C07D 471/20

(54) **SPIRO[HETEROCYCLE-IMIDAZO[1,2-a]INDENO[1,2-e]PYRAZINE]-4'-ONES, LEUR PREPARATION ET LES MEDICAMENTS LES CONTENANT**
SPIRO[HETEROZYCLISCHE-IMIDAZO[1,2-a]INDENO[1,2-e]PYRAZIN]-4'-ONE, IHRE HERSTELLUNG UND DIESE ENTHALTENDE ARZNEIMITTEL
SPIRO[HETEROCYCLE-IMIDAZO[1,2-a]INDENO[1,2-e]PYRAZINE]-4'-ONES, PREPARATION THEREOF AND DRUGS CONTAINING SAME

(30) Priorité: 02.11.1994 FR 9413060
(43) Date de publication de la demande: 20.08.1997
(73) Titulaire: RHONE-POULENC RORER S.A., 92160 Antony (FR)
(72) Inventeur: ALOUP, Jean-Claude, F-94290 Villeneuve-le-Roi (FR); AUDIAU, François, F-94220 Charenton-le-Pont (FR); BARREAU, Michel, F-91230 Montgeron (FR); DAMOUR, Dominique, F-75014 Paris (FR); GENEVOIS-BORELLA, Arielle, F-94320 Thiais (FR); JIMONET, Patrick, F-78450 Villepreux (FR); MIGNANI, Serge, F-92290 Châtenay-Malabry (FR); RIBEILL, Yves, F-91360 Villemoisson-sur-Orge (FR)
(74) Mandataire: Savina, Jacques
(86) Numéro de dépôt international: FR9501430
(87) Numéro de publication internationale: WO9614318

(56) Documents cités:
- WO-A-95/02601
- FR-A- 2 696 466

## Description

La présente invention concerne des composés de formule : 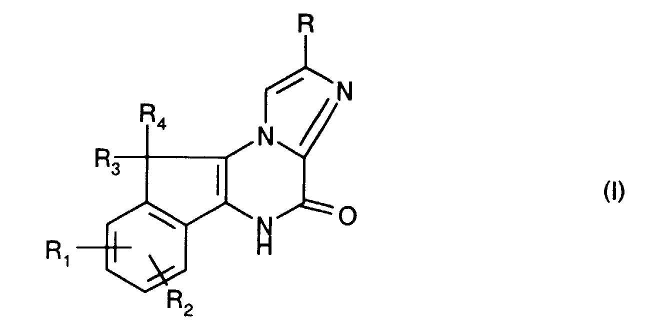 leurs sels, leur préparation et les médicaments les contenant.

Dans la formule (I)
- R représente un atome d'hydrogène ou un radical carboxy, alcoxycarbonyle ou carboxamido,
- R₁ et R₂, identiques ou différents, représentent des atomes d'hydrogène ou d'halogène ou des radicaux alkyle, alcoxy, amino, -N=CH-N(alk)alk', nitro, cyano, phényle, imidazolyle, SO₃H, hydroxy, polyfluoroalcoxy, carboxy, alcoxycarbonyle, -NH-CO-NR₅R₆, -N(alk)-CO-NR₅R₆, -N(alk-Ar)-CO-NR₅R₆, -NH-CS-NR₅R₆, -N(alk)-CS-NR₅R₆, -NH-CO-R₅, -NH-CS-R₇, -NH-C(=NR₉)-NR₈R₆, -N(alk)-C(=NR₉)-NR₈R₆, -CO-NR₈R₆, -NH-SO₂-NR₈R₆, -N(alk)-SO₂-NR₈R₆, -NH-SO₂-CF₃, -NH-SO₂-alk, -NH-SO₂-Ar, -NR₈R₁₀, -S(O)ₘ-alk-Ar, -SO₂-NR₈R₆ ou 2-oxo-1-imidazolidinyle dont la position -3 est éventuellement substituée par un radical alkyle ou 2-oxo-1-perhydropyrimidinyle dont la position -3 est éventuellement substituée par un radical alkyle,
- R₃ et R₄ forment ensemble avec l'atome de carbone auquel ils sont rattachés un cycle 2- ou 3-pyrrolidine, un cycle 2- ou 4-pipéridine ou un cycle 2-azacycloheptane, ces cycles étant éventuellement substitués sur l'azote par un radical alkyle, -CHO, -COOR₁₁, -CO-alk-COOR₆, -CO-alk-NR₆R₁₂, -CO-alk-CONR₆R₈, -CO-COOR₆, -CO-CH₂-O-CH₂-COOR₆, -CO-CH₂-S-CH₂-COOR₆, -CO-CH=CH-COOR₆, -CO-alk, -CO-Ar", -CO-alk-Ar", -CO-NH-Ar", -CO-NH-alk-Ar", -CO-Het, -CO-alk-Het, -CO-NH-Het, -CO-NH-alk-Het, -CO-NH₂, -CO-N H-alk, -CO-N(alk)alk', -CS-NH₂, -CS-NH-alk, -CS-NH-Ar", -CS-NH-Het, -alk-Het, -alk-NR₆R₈, -alk-COOR₆, -alk-CO-NR₆R₈, -alk-Ar", -SO₂-alk, -SO₂-Ar ou -CO-cycloalkyle dont le cycloalkyle est éventuellement substitué en -2 par un radical carboxy,
- R₅ représente un atome d'hydrogène ou un radical alkyle (1-9C en chaîne droite ou ramifiée), -alk-COOR₈, -alk-Het, -alk-NR₆R₈, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, carboxy, alcoxycarbonyle, cyano et -alk-COOR₈, phényle éventuellement substitué par un ou plusieurs substituants choisis parmi les atomes d'halogène et les radicaux alkyle, alcoxy, nitro, amino, hydroxy, -alk-NH₂, carboxy, alcoxycarbonyle, cyano et -alk-COOR₈ ou -Het,
- R₆ représente un atome d'hydrogène ou un radical alkyle,
- R₇ représente un radical alkyle ou phényle,
- R₈ représente un atome d'hydrogène ou un radical alkyle,
- R₉ représente un atome d'hydrogène ou un radical alkyle,
- R₁₀ représente un radical alkyle, Het ou alcoxycarbonyle,
- R₁₁ représente un radical alkyle ou phénylalkyle,
- R₁₂ représente un atome d'hydrogène ou un radical alkyle,
- alk représente un radical alkyle ou alkylène,
- alk' représente un radical alkyle,
- m est égal à 0, 1 ou 2,
- Ar représente un radical phényle,
- Ar" est un radical phényle ou phényle substitué par un ou plusieurs substituants choisis parmi les atomes d'halogène et les radicaux alkyle, alcoxy, nitro, amino, hydroxy, cyano, -alk-NH₂, COOR₆ et -alk-COOR₆,
- Het représente (a) un hétérocycle mono ou polycyclique saturé ou insaturé contenant 1 à 9 atomes de carbone et un ou plusieurs hétéroatomes (O, S, N) éventuellement substitué par un ou plusieurs radicaux alkyle, phényle, carboxy ou phénylalkyle.

Sauf mention contraire, dans les définitions qui précédent et celles qui suivent, les radicaux et portions alkyle, alkylène et alcoxy contiennent 1 à 6 atomes de carbone et sont en chaîne droite ou ramifiée, les radicaux cycloalkyle contiennent 3 à 6 atomes de carbone et les atomes d'halogène sont choisis parmi le fluor, le chlore, le brome et l'iode.

De préférence, Het est choisi parmi les cycles pyrrolyle éventuellement substitué par un ou plusieurs radicaux alkyle, phényle, carboxy ou phénylalkyle, pyridyle éventuellement substitué par un ou plusieurs radicaux alkyle, phényle, carboxy ou phénylalkyle, pyrimidinyle éventuellement substitué par un ou plusieurs radicaux alkyle, phényle, carboxy ou phénylalkyle, imidazolyle éventuellement substitué par un ou plusieurs radicaux alkyle, phényle, carboxy ou phénylalkyle, thiazolyle éventuellement substitué par un ou plusieurs radicaux alkyle, phényle, carboxy ou phénylalkyle, oxazolinyle éventuellement substitué par un ou plusieurs radicaux alkyle, phényle, carboxy ou phénylalkyle, thiazolinyle éventuellement substitué par un ou plusieurs radicaux alkyle, phényle, carboxy ou phénylalkyle, pyrazinyle éventuellement substitué par un ou plusieurs radicaux alkyle, phényle, carboxy ou phénylalkyle, tétrazolyle éventuellement substitué par un ou plusieurs radicaux alkyle, phényle, carboxy ou phénylalkyle ou triazolyle éventuellement substitué par un ou plusieurs radicaux alkyle, phényle, carboxy ou phénylalkyle. Les substituants préférés sont les radicaux méthyle, phényle, carboxy et benzyle.

Les énantiomères et diastéréoisomères des composés de formule (I) pour lesquels R₃ et R₄ forment ensemble avec l'atome de carbone auquel ils sont rattachés un cycle 2- ou 3-pyrrolidine, 2-pipéridine ou 2-azacycloheptane font également partie de l'invention ainsi que les isomères cis et trans des composés de formule (I) comportant un radical -CO-CH=CH-COOR₆.

Les composés de formule (I) pour lesquels R₃ et R₄ forment avec l'atome de carbone auquel ils sont rattachés un cycle 2-pyrrolidine, 2-pipéridine ou 2-azacycloheptane peuvent être préparés par action d'un dérivé de formule : 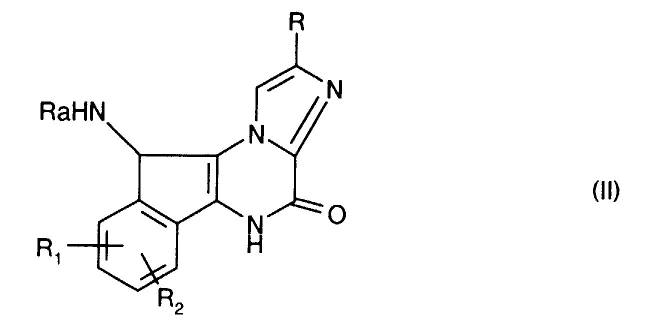 dans laquelle Ra représente un groupe protecteur de la fonction amine tel qu'un radical tert-butoxycarbonyle, R, R₁ et R₂ ont les mêmes significations que dans la formule (I), sur un dérivé de formule :

Hal-(CH₂)ₚ-Hal' (III)

dans laquelle Hal et Hal', identiques ou différents, sont des atomes d'halogène (chlore et brome de préférence) et p est égal à 3, 4 ou 5 puis déprotection du NH.

La condensation s'effectue au sein d'un solvant inerte tel que le diméthylsulfoxyde, en présence d'un hydrure de métal alcalin (hydrure de sodium par exemple), à une température voisine de 20°C.

La déprotection du NH se fait par toute méthode connue de l'homme de l'art comme celles décrites par W. GREENE et coll., "Protecting Groups in Organic Synthesis", second edition, 1991, John Wiley & Sons. En particulier, lorsque le groupe protecteur est un radical tert-butoxycarbonyle on opère au moyen d'acide trifluoroacétique à une température voisine de 20°C.

Les dérivés de formule (Il) peuvent être obtenus par protection de la fonction amine des dérivés correspondants pour lesquels Ra représente un atome d'hydrogène.

Cette protection s'effectue par tous les procédés connus de protection d'une fonction amine comme ceux décrits par W. GREENE et coll., "Protecting Groups in Organic Synthesis, second edition, 1991, John Wiley & Sons. Dans le cas où Ra représente un radical tert-butoxycarbonyle on fait réagir de préférence le carbonate de di-tert-butyle, au sein d'un solvant inerte tel que le diméthylformamide, à une température voisine de 20°C.

Les dérivés de formule (Il) pour lesquels Ra représente un atome d'hydrogène peuvent être obtenus par hydrolyse des composés de formule (Il) correspondants pour lesquels Ra représente un radical alkylcarbonyle, suivie pour les composés pour lesquels R représente un radical alcoxycarbonyle d'une estérification de l'acide correspondant.

Cette hydrolyse s'effectue généralement au moyen d'un acide tel que l'acide chlorhydrique, en milieu aqueux, à la température d'ébullition du milieu réactionnel. L'estérification s'effectue au moyen d'un alcool (C1-C6 en chaîne droite ou ramifiée), en milieu acide (acide chlorhydrique ou sulfurique par exemple), à la température d'ébullition du milieu réactionnel.

Les dérivés de formule (II) pour lesquels Ra représente un radical alkylcarbonyle peuvent être obtenus par réduction d'un dérivé de formule : 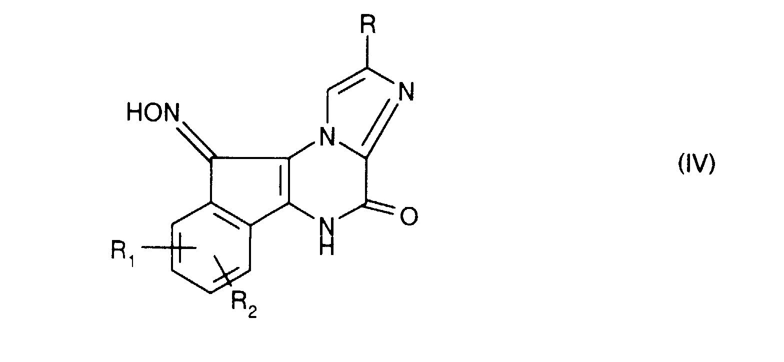 dans laquelle R, R₁ et R₂ ont les mêmes significations que dans la formule (I), en présence d'un acide alk-COOH, suivie de l'action de l'anhydride d'acide (alkCO)₂O dans lesquels alk représente un radical alkyle.

La réduction s'effectue généralement à une température comprise entre 50 et 100°C. Comme agent réducteur on utilise de préférence le zinc. La réaction avec l'anhydride s'effectue de préférence à une température voisine de 20°C.

Les dérivés de formule (IV) peuvent être obtenus par action d'un nitrite d'alkyle sur un dérivé de formule : 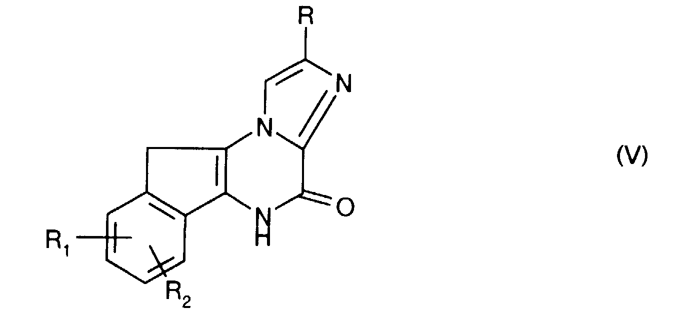 dans laquelle R, R₁ et R₂ ont les mêmes significations que dans la formule (I).

Cette réaction s'effectue de préférence au sein d'un solvant inerte tel que le diméthylsulfoxyde, en présence d'un hydrure de métal alcalin tel que l'hydrure de sodium, à une température voisine de 20°C. De préférence on utilise le nitrite d'isoamyle.

Les dérivés de formule (V) pour lesquels R représente un atome d'hydrogène peuvent être obtenus par désalkylation et désalification d'un dérivé de formule : 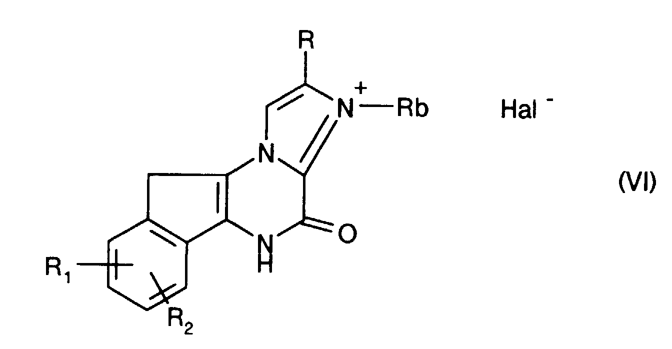 dans laquelle R représente un atome d'hydrogène, R₁ et R₂ ont les mêmes significations que dans la formule (I), Rb représente un radical alkyle et Hal représente un atome d'halogène (brome de préférence).

Cette réaction s'effectue de préférence en présence d'imidazole, à une température comprise entre 100 et 200°C.

Les dérivés de formule (VI) peuvent être obtenus par action d'un dérivé de formule : 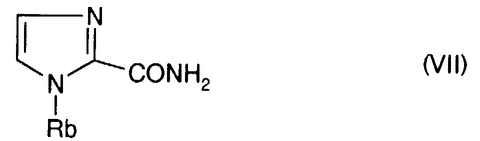 dans laquelle Rb a les mêmes significations que dans la formule (VI), sur une halogénoindanone de formule : 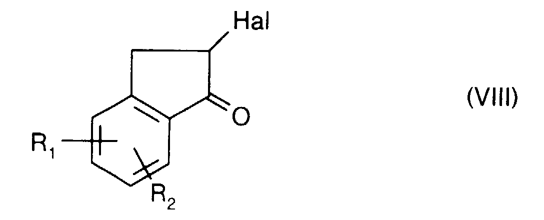 dans laquelle R₁ et R₂ ont les mêmes significations que dans la formule (I) et Hal représente un atome d'halogène.

Cette réaction s'effectue généralement au sein d'un solvant inerte tel que le diméthylformamide, à une température comprise entre 50 et 150°C et de préférence à 115°C.

Les dérivés de formule (VII) peuvent être obtenus par application ou adaptation de la méthode décrite par D.D. DAVEY, J. Org. Chem., 52, 4379 (1987).

Les dérivés de formule (VIII) peuvent être obtenus par application ou adaptation de la méthode décrite par OLIVIER et coll., Bull. Soc. Chim. France, 3092 (1973) et dans le brevet DE 2640358.

Les dérivés de formule (V) pour lesquels R représente un radical alcoxycarbonyle à l'exception de tert-butoxycarbonyle peuvent être obtenus par cyclisation d'un dérivé de formule : 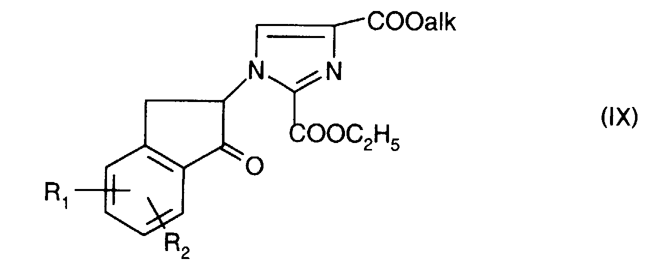 dans laquelle R₁ et R₂ ont les mêmes significations que dans la formule (I) et alk représente un radical alkyle sauf tert-butyle.

Cette cyclisation s'effectue généralement au sein de l'acide acétique, en présence d'acétate d'ammonium, à la température d'ébullition du milieu réactionnel.

Les dérivés de formule (IX) peuvent être obtenus par action d'un 4-alcoxycarbonyle-imidazole-2-carboxylate d'éthyle sur un dérivé de formule (VIII) dans laquelle R₁ et R₂ ont les mêmes significations que dans la formule (I) et Hal représente un atome d'halogène.

Cette réaction s'effectue généralement au sein d'un solvant inerte tel qu'un alcool (méthanol, éthanol par exemple), une cétone (acétone par exemple), un hydrocarbure aromatique (toluène par exemple), le diméthylformamide ou en absence de solvant, éventuellement en présence d'une base organique telle que l'hydrure de sodium ou le carbonate de potassium, à une température comprise entre 20°C et la température d'ébullition du milieu réactionnel ou la fusion du milieu réactionnel.

Les 4-alcoxycarbonyle-imidazole-2-carboxylate d'éthyle peuvent être obtenus par application ou adaptation de la méthode décrite par P.S. BRANCO et coll., Tetrahedron, 48 (30), 6335 (1992).

Les dérivés de formule (V) pour lesquels R représente un radical tert-butoxycarbonyle peuvent être obtenus par action d'isobutène sur un dérivé de formule (V) correspondant pour lequel R représente un radical carboxy.

Cette réaction s'effectue généralement au sein d'un solvant inerte tel que le dichlorométhane, le dioxane, en présence d'acide sulfurique concentré, à une température comprise entre 20°C et la température d'ébullition du milieu réactionnel.

Les dérivés de formule (V) pour lesquels R représente un radical carboxy peuvent être obtenus par cyclisation d'un dérivé de formule (IX) pour lequel alk représente un radical tert-butyle.

Cette cyclisation s'effectue généralement au sein de l'acide acétique, en présence d'acétate d'ammonium, à la température d'ébullition du milieu réactionnel.

Les dérivés de formule (V) pour lesquels R représente un radical carboxamido peuvent être obtenus par cyclisation d'un dérivé de formule : 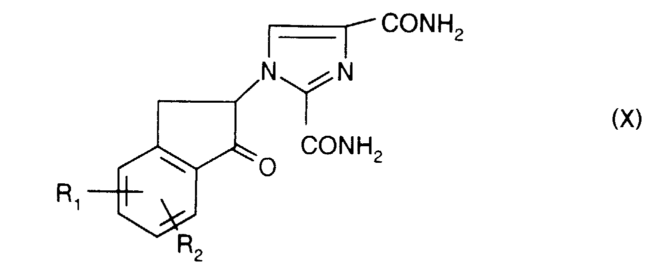 dans laquelle R₁ et R₂ ont les mêmes significations que dans la formule (I). Cette cyclisation s'effectue généralement au moyen d'un acide tel que l'acide chlorhydrique ou l'acide acétique, en solution aqueuse, à une température comprise entre 20°C et la température d'ébullition du milieu réactionnel.

Les dérivés de formule (X) peuvent être obtenus par action d'ammoniac sur un dérivé de formule (IX) correspondant.

Cette réaction s'effectue généralement au sein d'un solvant inerte tel qu'un alcool éventuellement en autoclave, à une température comprise entre -30°C et 100°C.

Les composés de formule (I) pour lesquels R₃ et R₄ forment avec l'atome de carbone auquel ils sont rattachés un cycle 3-pyrrolidine peuvent être préparés par condensation de N-n-butoxyméthyl-N-triméthylsilylméthyl-benzylamine sur un dérivé de formule : 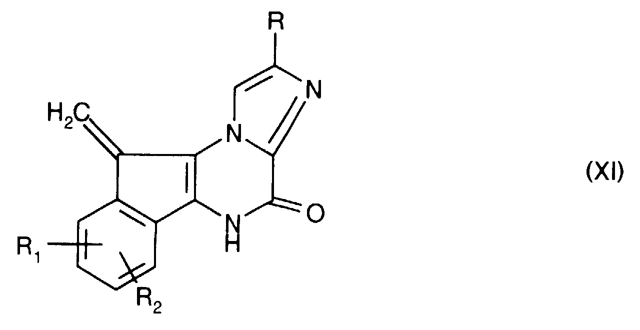 dans laquelle R, R₁ et R₂ ont les mêmes significations que dans la formule (I), suivie d'une débenzylation du NH.

La condensation s'effectue au sein d'un solvant inerte tel que le diméthylformamide, en présence d'une quantité catalytique d'acide trifluoroacétique, à une température comprise entre 15 et 70°C. La débenzylation s'effectue généralement au moyen d'hydrogène, au sein d'un solvant inerte (diméthylformamide, acide acétique par exemple), en présence d'un catalyseur d'hydrogénation tel que le charbon palladié, l'hydroxyde de palladium ou le palladium, sous une pression d'hydrogène comprise entre 1 et 20 bar, à une température comprise entre 20 et 50°C.

Le N-n-butoxyméthyl-N-triméthylsilylméthyl-benzylamine peut être obtenu par la méthode décrite par Y. TERAO et coll., Chem. Pharm. Bull., 33, 2762 (1985).

Les dérivés de formule (XI) peuvent être obtenus par application ou adaptation de la méthode décrite dans les exemples.

Les composés de formule (I) pour lesquels R₃ et R₄ forment avec l'atome d'azote auquel ils sont attachés un cycle 4-pipéridine peuvent être préparés par action de N,N-bis(2-chloroéthyl)p-toluènesulfonamide sur un dérivé de formule (V) dans laquelle R, R₁ et R₂ ont les mêmes significations que dans la formule (I), suivie d'une hydrolyse de la fonction sulfonamide.

Cette réaction s'effectue de préférence au sein d'un solvant inerte tel que le diméthylsulfoxyde, en présence d'un hydrure de métal alcalin (hydrure de sodium par exemple), à une température voisine de 20°C. L'hydrolyse de la fonction sulfonamide s'effectue de préférence au sein d'un acide tel que l'acide bromhydrique, à la température d'ébullition du milieu réactionnel.

Les composés de formule (I) pour lesquels R₃ et R₄ forment avec l'atome de carbone auquel ils sont rattachés un cycle 2- ou 3-pyrrolidine, 2- ou 4-pipéridine ou 2-azacycloheptane dont l'atome d'azote est substitué par un radical alkyle (1C) peuvent être préparés par action de formaldéhyde et d'acide formique sur un composé de formule (I) correspondant pour lequel R₃ et R₄ forment avec l'atome de carbone auquel ils sont rattachés un cycle 2- ou 3-pyrrolidine, 2- ou 4-pipéridine ou 2-azacycloheptane.

Cette réaction s'effectue à une température comprise entre 20 et 35°C.

Les composés de formule (I) pour lesquels R₃ et R₄ forment avec l'atome de carbone auquel ils sont rattachés un cycle 2- ou 3-pyrrolidine, 2- ou 4-pipéridine ou 2-azacycloheptane dont l'atome d'azote est substitué par un radical alkyle (2-6C) peuvent être préparés par action d'un acide alk-COOH dans lequel alk représente un radical alkyle 1-5C sur un composé de formule (I) correspondant pour lequel R₃ et R₄ forment avec l'atome de carbone auquel ils sont rattachés un cycle 2- ou 3-pyrrolidine, 2- ou 4-pipéridine ou 2-azacycloheptane, en présence de borohydrure de sodium.

Cette réaction s'effectue en présence d'un excès de l'acide alk-COOH qui sert de solvant, à une température voisine de 45°C.

Les composés de formule (I) pour lesquels R₃ et R₄ forment avec l'atome de carbone auquel ils sont rattachés un cycle 2- ou 3-pyrrolidine, 2- ou 4-pipéridine ou 2-azacycloheptane dont l'atome d'azote est substitué par un radical -CO-alk-NR₆R₁₂ dans lequel R₆ et R₁₂ représentent chacun un atome d'hydrogène peuvent être préparés par action d'un composé de formule (I) correspondant pour lequel R₃ et R₄ forment avec l'atome de carbone auquel ils sont rattachés un cycle 2- ou 3-pyrrolidine, 2- ou 4-pipéridine ou 2-azacycloheptane sur un dérivé de formule : 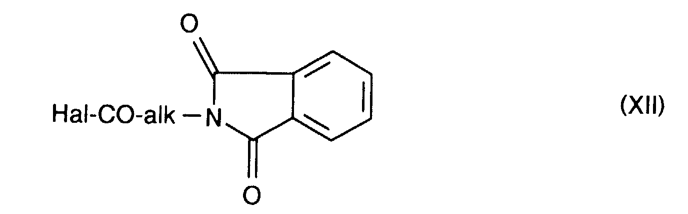 dans laquelle Hal représente un atome d'halogène et alk représente un radical alkyle puis déprotection du NH₂.

Cette réaction s'effectue généralement au sein d'un solvant inerte tel que le diméthylformamide, en présence d'un accepteur d'acide tel qu'une base organique azotée (pyridine, trialkylamine comme la triéthylamine par exemple), à une température comprise entre 0°C et la température d'ébullition du milieu réactionnel. La déprotection s'effectue au sein d'un alcool aliphatique inférieur (éthanol par exemple), en présence d'hydrazine, à la température d'ébullition du milieu réactionnel.

Les dérivés de formule (XII) peuvent être obtenus par application ou adaptation de la méthode décrite par K. BALENOVIC et coll., J. Org. Chem., 17, 1149 (1952).

Les composés de formule (I) pour lesquels R₃ et R₄ forment avec l'atome de carbone auquel ils sont rattachés un cycle 2- ou 3-pyrrolidine, 2- ou 4-pipéridine ou 2-azacycloheptane dont l'atome d'azote est substitué par un radical alkyle (2-6C), -COOR₁₁, -alk-Het, -alk-NR₆R₈, -alk-COOR₆, -alk-Ar", -CO-alk, -CO-alk-COOR₆, -CO-alk-NR₆R₁₂, -CO-COOR₆, -CO-cycloalkyle dont le cycloalkyle est éventuellement substitué en -2 par un radical carboxy, -CO-Ar", -CO-alk-Ar", -CO-Het, -CO-alk-Het, -CO-N(alk)alk', -SO₂-alk ou -SO₂-Ar peuvent être préparés par action d'un composé de formule (I) correspondant pour lequel R₃ et R₄ forment avec l'atome de carbone auquel ils sont rattachés un cycle 2- ou 3-pyrrolidine, 2- ou 4-pipéridine ou 2-azacycloheptane sur un halogénure Hal-Rc dans lequel Hal représente un atome d'halogène et Rc représente un radical alkyle (2-6C), -COOR₁₁, -alk-Het, -alk-NR₆R₈, -alk-COOR₆, -alk-Ar", -CO-alk, -CO-alk-COOR₆, -CO-alk-NR₆R₁₂, -CO-COOR₆, -CO-cycloalkyle(3-6C) dont le cycloalkyle est éventuellement substitué en -2 par un radical carboxy, -CO-Ar", -CO-alk-Ar", -CO-Het, -CO-alk-Het, -CO-N(alk)alk', -SO₂-alk ou -SO₂-Ar, alk, alk', Het, R₆, R₈, R₁₁, R₁₂, Ar et Ar" ayant les mêmes significations que dans la formule (I).

Cette réaction s'effectue généralement au sein d'un solvant inerte tel que le diméthylformamide, en présence d'un accepteur d'acide tel qu'un carbonate de métal alcalin (carbonate de sodium ou de potassium par exemple), une trialkylamine (triéthylamine par exemple) ou la pyridine, à une température comprise entre 0°C et la température d'ébullition du milieu réactionnel.

Les halogénures Hal-Rc sont commercialisés ou peuvent être préparés selon les méthodes suivantes :
- ceux pour lesquels Rc représente un radical -alk-NR₆R₈ peuvent être obtenus par action de l'amine HNR₆R₈ dans laquelle R₆ et R₈ ont les mêmes significations que dans la formule (I) sur un halogénure Hal-alk-Hal dans lequel Hal représente un atome d'halogène et alk représente un radical alkyle, au sein d'un solvant inerte tel que le diméthylformamide, en présence d'un accepteur d'acide tel qu'une base azotée, à une température comprise entre 0 et 25°C,
- ceux pour lesquels Rc représente un radical -alk-COOR₆ peuvent être obtenus par action d'un dérivé Hal-alk-Hal dans lequel Hal représente un atome d'halogène et alk représente un radical alkyle sur un cyanure alcalin (cyanure de sodium ou de potassium), au sein d'un mélange eau-alcool, à une température comprise entre 0°C et la température d'ébullition du milieu réactionnel, suivie de l'action d'un acide fort tel que l'acide chlorhydrique, éventuellement en présence d'un alcool aliphatique inférieur, à une température comprise entre 0°C et la température d'ébullition du milieu réactionnel,
- ceux pour lesquels Rc représente un radical -CO-alk, -CO-alk-COOR₆, -CO-alk-NR₆R₁₂, -CO-COOR₆, -CO-cycloalkyle, -CO-Ar", -CO-alk-Ar", -CO-Het, -CO-alk-Het peuvent être obtenus par chauffage d'un acide correspondant HO-Rc dans lequel Rc est défini comme précédemment et d'un réactif d'halogénation tel que le chlorure de thionyle, le bromure de thionyle, un halogénure de phosphore (PCl₅, POCl₃ par exemple), au sein d'un solvant inerte tel qu'un solvant chloré (1,2-dichloroéthane par exemple), à une température comprise entre 20°C et la température d'ébullition du milieu réactionnel. Les acides correspondants sont commercialisés ou les acides HOOC-alk-NR₆R₁₂ peuvent être obtenus par action d'un halogénure alkOOC-alk-Hal dans lequel Hal représente un atome d'halogène et alk un radical alkyle, sur une amine HNR₆R₁₂ dans laquelle R₆ et R₁₂ ont les mêmes significations que dans la formule (I), en présence d'un carbonate de métal alcalin ou une trialkylamine, à une température comprise entre 20°C et la température d'ébullition du milieu réactionnel, suivie d'une hydrolyse en milieu basique (soude-alcool), à une température comprise entre 0 et 60°C et d'une acidification au moyen d'un acide (HCI par exemple), à une température comprise entre 20 et 60°C. Les acides HOOC-Het, HOOC-alk-Het, HOOC-Ar" et HOOC-alk-Ar" peuvent être obtenus à partir de l'hétérocycle et du benzène éventuellement substitué correspondant par application ou adaptation des méthodes décrites par L. ESTEL et coll., J. Heterocyclic Chem., 26, 105 (1989); N.S. NARASIMHAN et coll., Synthesis, 957 (1983); A. TURCK et coll., Synthesis, 881 (1988), A.J. CLARKE et coll., Tetrahedron Lett., 27, 2373 (1974); A.R. KATRITZKY et coll., Org. Perp. Procedure Int., 20 (6), 585 (1988); N. FURUKAWA et coll., Tetrahedron Lett., 28 (47), 5845 (1987); H.W. GSCHWEND et coll., Organic Reactions, 26, 1 (1979) et V. SNIECKUS, Chem. Rev., 90, 879 (1990). De préférence, on prépare le dérivé organométallique correspondant de l'hétérocycle ou du benzène éventuellement substitué (organolithien, organomagnésien par exemple) et le fait réagir soit sur du CO₂ soit sur un dérivé Hal-alk-COOalk dans lequel Hal représente un atome d'halogène et alk représente un radical alkyle suivie d'une réaction d'hydrolyse laquelle s'effectue généralement au moyen d'une base telle qu'un hydroxyde de métal alcalin (soude, potasse par exemple) au sein d'un mélange eau-alcool aliphatique inférieur, à une température comprise entre 20 et 80°C. Les dérivés Hal-alk-COOalk sont commercialisés ou peuvent être obtenus par action de Hal-alk-Hal dans lequel Hal représente un atome d'halogène et alk représente un radical alkyle sur un cyanure alcalin tel que le cyanure de sodium ou de potassium, au sein d'un mélange eau-alcool aliphatique inférieur, à une température comprise entre 0°C et la température d'ébullition du milieu réactionnel suivie de l'action d'un acide tel que l'acide chlorhydrique, en présence d'un alcool, à une température comprise entre 0°C et la température d'ébullition du milieu réactionnel. Les acides HOOC-alk-COOR₆ peuvent être obtenus à partir des diacides correspondants par application ou adaptation de la méthode décrite par D. REHN et coll., J. Chem. Research (s), 119 (1977),
- ceux pour lesquels Rc représente un radical -alk-Het ou -alk-Ar" peuvent être obtenus à partie des alcools correspondants HO-Rc dans lequel Rc est défini comme précédemment par application ou adaptation des méthodes décrites par R.C. LAROCK, "Comprehensive Organic Transformations", ed. VCH, page 353 (1989). Les alcools correspondants HO-alk-Het et HO-alk-Ar" sont commercialisés ou peuvent être obtenus à partir des dérivés organométalliques correspondants par application ou adaptation des méthodes décrites par L. ESTEL et coll., J. Heterocyclic Chem., 26, 105 (1989); N.S. NARASIMHAN et coll., Synthesis, 957 (1983) et Tetrahedron Lett., 22 (29), 2797 (1981); H.W. GSCHWEND et coll., Organic Reactions, 26, 1 (1979), V. SNIECKUS, Chem. Rev., 90, 879 (1990) et F. MARCHAIS et coll., J. Heterocyclic Chem, 25, 81 (1988). De préférence, on fait réagir l'organolithien ou l'organomagnésien de l'hétérocycle ou du benzène éventuellement substitué sur le formol, un aldéhyde, une cétone, un époxyde ou Hal-alk-OP où P est un groupe protecteur (méthyléther, tétrahydropyranyléther, benzyléther ou triéthylsilyléther par exemple) puis on libère la fonction alcool par application ou adaptation des méthodes décrites par W. GREENE et coll., "Protecting Groups in Organic Synthesis, second edition, 1991, John Wiley & Sons. Les alcools correspondants HO-alk-Het et HO-alk-Ar" peuvent aussi être obtenus par réduction des acides carboxyliques ou des esters correspondants au moyen d'hydrure de lithium et d'aluminium, au sein d'un solvant inerte tel que le tétrahydrofuranne ou le diéthyléther, à la température d'ébullition du milieu réactionnel. Les alcools HO-alk(2-6C)-Het peuvent aussi être obtenus par application ou adaptation des la méthode décrite par J.T. MEYER et coll., Helv. Chem. Acta, 65, 1868 (1982) à partir des dérivés Hal-alk(1-5C)-Het dans lesquels Hal représente un atome d'halogène et alk représente un radical alkyle, qui sont eux-mêmes obtenus par action d'un agent d'halogénation (dérivé halogéné du phosphore ou chlorure de thionyle) sur un dérivé HO-alk(1-5C)-Het correspondant, éventuellement au sein d'un solvant inerte tel que le dichlorométhane, à une température comprise entre 20 et 40°C,
- ceux pour lesquels Rc représente un radical -SO₂-alk ou -SO₂-Ar peuvent être préparés par halogénation des acides sulfoniques correspondants au moyen d'un dérivé halogéné du phosphore ou de chlorure de thionyle, au sein d'un solvant inerte tel que le dichlorométhane, à une température comprise entre 20 et 40°C,
- ceux pour lesquels Rc représente un radical -COOR₁₁ peuvent être obtenus par application ou adaptation des méthodes décrites dans HOUBEN WEYL, band 8, page 102 (1952).

Les composés de formule (I) pour lesquels R₃ et R₄ forment avec l'atome de carbone auquel ils sont rattachés un cycle 2- ou 3-pyrrolidine, 2- ou 4-pipéridine ou 2-azacycloheptane dont l'atome d'azote est substitué par un radical -CO-alk-CONR₆R₈ ou -alk-CO-NR₆R₈ peuvent être préparés par action d'un composé de formule (I) correspondant pour lequel R₃ et R₄ forment avec l'atome de carbone auquel ils sont rattachés un cycle 2- ou 3-pyrrolidine, 2- ou 4-pipéridine ou 2-azacycloheptane dont l'atome d'azote est substitué par un radical -CO-alk-COOR₆ ou -alk-COOR₆ sur une amine HNR₆R₈ dans laquelle R₆ et R₈ ont les mêmes significations que dans la formule (I).

Lorsque l'on met en oeuvre l'acide, on opère en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxane par exemple), un amide (diméthylformamide) ou un solvant chloré (chlorure de méthylène, dichloro-1,2 éthane, chloroforme par exemple) à une température comprise entre 0°C et la température de reflux du mélange réactionnel. Lorsque l'on met en oeuvre un ester, on opère alors soit en milieu organique, éventuellement en présence d'un accepteur d'acide tel qu'une base organique azotée (trialkylamine, pyridine, diaza-1,8 bicyclo[5.4.0]undécène-7 ou diaza-1,5 bicyclo[4.3.0]nonène-5 par exemple), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0°C et la température d'ébullition du milieu réactionnel.

Les composés de formule (I) pour lesquels R₃ et R₄ forment avec l'atome de carbone auquel ils sont rattachés un cycle 2- ou 3-pyrrolidine, 2- ou 4-pipéridine ou 2-azacycloheptane dont l'atome d'azote est substitué par un radical -CHO peuvent être préparés par action d'un composé de formule (I) correspondant pour lequel R₃ et R₄ forment avec l'atome de carbone auquel ils sont rattachés un cycle 2- ou 3-pyrrolidine, 2- ou 4-pipéridine ou 2-azacycloheptane sur CH₃COOCHO.

Cette réaction s'effectue de préférence au sein d'un solvant inerte tel que l'acide formique, en présence d'acétate de sodium, à une température voisine de 20°C.

Les composés de formule (I) pour lesquels R₃ et R₄ forment avec l'atome de carbone auquel ils sont rattachés un cycle 2- ou 3-pyrrolidine, 2- ou 4-pipéridine ou 2-azacycloheptane dont l'atome d'azote est substitué par un radical -CO-Het, -CO-alk-COOR₆, -CO-COOR₆, -CO-alk-NR₆R₁₂, -CO-Ar", -CO-alk-Ar", -CO-alk-Het, -CO-alk ou -CO-cycloalkyle dont le cycloalkyle est éventuellement substitué en -2 par un radical carboxy peuvent également être préparés par action d'un composé de formule (I) correspondant pour lequel R₃ et R₄ forment avec l'atome de carbone auquel ils sont rattachés un cycle 2- ou 3-pyrrolidine, 2- ou 4-pipéridine ou 2-azacycloheptane sur un dérivé HO-Rd dans lequel Rd représente un radical -CO-Het, -CO-alk-COOR₆, -CO-COOR₆, -CO-alk-NR₆R₁₂, -CO-Ar", -CO-alk-Ar", -CO-alk-Het, -CO-alk, -CO-cycloalkyle(3-6C) dont le cycloalkyle est éventuellement substitué en -2 par un radical carboxy, Het, alk, R₆, R₈, R₁₂, Het et Ar" ayant les mêmes significations que dans la formule (I).

Cette réaction s'effectue généralement au sein d'un solvant inerte tel que le diméthylformamide, en présence d'hydroxybenzotriazole ou de 1-(3-diméthylaminopropyl)-3-éthylcarbodiimide et d'une base organique telle qu'une trialkylamine (triéthylamine par exemple), à une température comprise entre 0 et 30°C.

Les composés de formule (I) pour lesquels R₃ et R₄ forment avec l'atome de carbone auquel ils sont rattachés un cycle 2- ou 3-pyrrolidine, 2- ou 4-pipéridine ou 2-azacycloheptane dont l'atome d'azote est substitué par un radical -CO-alk-COOR₆ dans lequel alk contient 1 à 3 atomes de carbone en chaîne droite, -CO-CH₂-C(CH₃)₂-CH₂-COOR₆, -CO-CH₂-CH₂-C(CH₃)₂-COOR₆, -CO-CH₂-C(CH₃)₂-COOR₆ -CO-CH₂-O-CH₂-COOR₆, -CO-CH₂-S-CH₂-COOR₆, -CO-CH=CH-COOR₆, -CO-cycloalkyle (6C) dont le cycloalkyle est substitué en -2 par un radical carboxy, -CO-Ar" dans lequel Ar" représente un radical phényle substitué en -2 par un radical carboxy, -CO-Het dans lequel Het représente un radical 2-ou 4-pyridyle substitué en position -3 par un radical carboxy ou 3-pyridyle substitué en position -4 par un radical carboxy et R₆ représente un atome d'hydrogène peuvent être préparés par action d'un anhydride de formules : 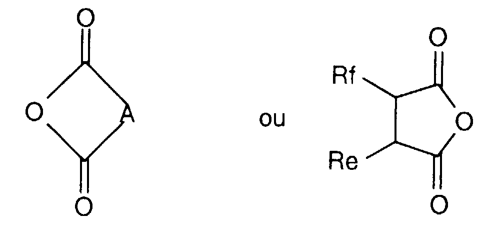 dans lesquelles A représente un radical alkyle (1-3C en chaîne droite), -CH₂-C(CH₃)₂-CH₂-, -CH₂-CH₂-C(CH₃)₂-, -CH₂-C(CH₃)₂-, -CH₂-O-CH₂-, -CH₂-S-CH₂, -CH=CH-, Re et Rf forment ensemble avec les 2 atomes de carbone auxquels ils sont rattachés un radical cycloalkyle(6C), phényle ou pyridyle sur un composé de formule (I) correspondant pour lesquels R₃ et R₄ forment avec l'atome de carbone auquel ils sont rattachés un cycle 2- ou 3-pyrrolidine, 2- ou 4-pipéridine ou 2-azacycloheptane.

Cette réaction s'effectue généralement au sein d'un solvant inerte tel que l'acide acétique, à une température voisine de 20°C ou en présence de 4-diméthylaminopyridine, au sein du dioxane, à la température d'ébullition du milieu réactionnel.

Les composés de formule (I) pour lesquels R₃ et R₄ forment avec l'atome de carbone auquel ils sont rattachés un cycle 2- ou 3-pyrrolidine, 2- ou 4-pipéridine ou 2-azacycloheptane dont l'atome d'azote est substitué par un radical -CO-NH-alk-Ar", -CO-NH-Het, -CO-NH-alk-Het, -CO-NH-Ar", -CO-NH-alk, -CO-NH₂, -CSNH₂, -CS-NH-alk, -CS-NH-Ar" ou -CS-NH-Het peuvent être préparés par action d'un composé de formule (I) correspondant pour lequel R₃ et R₄ forment avec l'atome de carbone auquel ils sont rattachés un cycle 2- ou 3-pyrrolidine, 2- ou 4-pipéridine ou 2-azacycloheptane sur un dérivé de formule Rg=C=N-Rh dans lequel Rg représente un atome d'oxygène ou de soufre et Rh représente un radical triméthylsilyle, alkyle, Het, -alk-Ar", -alk-Het ou Ar", Het, alk et Ar" ayant les mêmes significations que dans la formule (I).

Cette réaction s'effectue de préférence au sein d'un solvant inerte tel que le diméthylformamide, le tétrahydrofuranne ou le dioxane, à une température comprise entre 20°C et la température d'ébullition du milieu réactionnel, suivie éventuellement d'une hydrolyse du dérivé silylé précédemment obtenu au moyen d'une solution aqueuse, à une température comprise entre 20 et 50°C.

Les dérivés O=C=N-Rh sont commercialisés ou peuvent être obtenus par action de phosgène sur l'amine primaire correspondante H₂N-Rh, par adaptation des méthodes décrites par R.L. SHRINER et coll., Organic Synth., II, 453; G.M. DYON, Organic Synth., I, 165; R.J. SLOCOMPIE et coll., J. Am. Chem. Soc., 72, 1888 (1950) et S. PATAI, "The chemistry of cyanates and their thio derivatives", Ed. John Wiley and Sons, page 619 (1977). Les amines primaires correspondantes sont commercialisées ou celles pour lesquelles Rh représente un radical Het ou Ar" peuvent être obtenues par application ou adaptation des méthodes décrites par B.A. TERTOV et coll., Khim. Geterotsikl. Soedin, II, 1552 (1972) et R.C. LAROCK, "Comprehensive Organic Transformations", Ed. VCH, page 399, qui consiste à faire réagir l'organolithien ou l'organomagnésien de l'hétérocycle ou du benzène éventuellement substitué sur PhN₃, en présence d'acide acétique, de NH₂OCH₃, (PHO)₂PON₃ ou de N₃CH₂Si(CH₃)₃. Les organolithiens ou organomagnésiens peuvent être obtenus par application ou adaptation des méthodes décrites par D.L. COMINS et coll., J. Org. Chem., 52, 104 (1987); N. FURUKANA et coll., Tetrahedron Lett., 28 (47), 5845 (1987); A.R. KATRITZKY et coll., Org. Prep. Procedure Int., 20 (6), 585 (1988); A.J. CLARKE et coll., Tetrahedron Lett., 27, 2373 (1974) et A.W. GSCHWEN et coll., Organic Reaction, 26, 1 (1979). Les amines pour lesquelles Rh représente un radical -alk-Het, -alk-Ar" sont commercialisées ou sont obtenues à partir des halogénures correspondants par action de NaN(SiCH₃)₃ ou du sel de potassium du phtalimide, au sein d'un solvant inerte tel que le diméthylformamide, en présence d'une base organique telle qu'une trialkylamine ou la pyridine, à une température comprise entre 0°C et la température d'ébullition du milieu réactionnel suivie d'une hydrolyse en milieu acide (HCI par exemple), à une température comprise entre 20°C et la température d'ébullition du milieu réactionnel. Les amines pour lesquelles Rh représente un radical -alk-Ar" peuvent également être obtenus par application ou adaptation des méthodes décrites par J.F. KING et coll., J. Am. Chem. Soc., 114, 3028 (1992); B.M. ADGER et coll., Tetrahedron Lett., 25 (45), 5219 (1984); R. SCARPATI et coll., Gazz. Chim. Ital., 97 (5), 654 (1967).

Les dérivés S=C=N-Rh peuvent être obtenus à partir des amines primaires correspondantes H₂N-Rh par action de thiophosgène par application ou adaptation des méthodes décrites par R.L. SHRINER et coll., Org. Synth., II, 453, G.M. DYSON, Organic Synth., I, 165, R.J. SLOCOMPIE et Coll., J. Am. Chem. Soc, 72, 1888 (1950) et S. PATAI, "The chemistry of cyanates and their thio derivatives", Ed. John WILEY and Sons, pages 619 et 819 (1977).

Les composés de formule (I) pour lesquels R représente un radical carboxy peuvent également être préparés par hydrolyse du composé de formule (I) correspondant pour lequel R représente un radical alcoxycarbonyle.

Cette hydrolyse s'effectue de préférence soit au moyen d'un acide minéral (acide chlorhydrique ou sulfurique par exemple), en solution aqueuse, à une température comprise entre 20°C et la température d'ébullition du milieu réactionnel; soit au moyen d'une base (soude, potasse, carbonate de potassium par exemple), en solution aqueuse ou en milieu hydroorganique (eau-tétrahydrofuranne, eau-dioxane par exemple), à une température comprise entre 0°C et la température d'ébullition du milieu réactionnel.

Les énantiomères des composés de formule (I) peuvent être obtenus par dédoublement des racémiques par exemple par chromatographie sur colonne chirale selon W.H. PIRCKLE et coll., asymetric synthesis, vol. 1, Academic Press (1983) ou par synthèse à partir des précurseurs chiraux.

Les isomères et diastéréoisomères des composés de formule (I) peuvent être séparés par les méthodes connues habituelles, par exemple par cristallisation ou chromatographie.

Il est entendu pour l'homme du métier que, pour la mise en oeuvre des procédés selon l'invention décrits précédemment, il peut être nécessaire d'introduire des groupes protecteurs des fonctions amino, hydroxy et carboxy afin d'éviter des réactions secondaires. Ces groupes sont ceux qui permettent d'être éliminés sans toucher au reste de la molécule. Comme exemples de groupes protecteurs de la fonction amino on peut citer les carbamates de tert-butyle ou de méthyle qui peuvent être régénérées au moyen d'iodotriméthylsilane. Comme exemples de groupes protecteurs de la fonction hydroxy, on peut citer les triéthylsilyle, benzyle. Comme groupes protecteurs des fonctions carboxy, on peut citer les esters (méthoxyméthylester, tétrahydropyranylester, benzylester par exemple, les oxazoles et les 2-alkyl-1,3-oxazolines. D'autres groupes protecteurs utilisables sont décrits par W. GREENE et coll., Protecting Groups in Organic Synthesis, second edition, 1991, John Wiley & Sons.

Les composés de formule (I) peuvent être purifiés par les méthodes connues habituelles, par exemple par cristallisation, chromatographie ou extraction.

Les composés de formule (I) comportant un reste basique peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) comportant un reste acide peuvent éventuellement être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'une amine ou d'un sel d'une amine sur un composé de formule (I), dans un solvant. Le sel formé est séparé par les méthodes habituelles.

Ces sels font également partie de l'invention.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, benzylamine, N-benzyl-p-phénéthylamine, choline, arginine, leucine, lysine, N-méthyl glucamine).

Les composés de formule (I) présentent des propriétés pharmacologiques intéressantes. Ces composés sont des antagonistes du récepteur de l'acide a-amino-3-hydroxy-5-méthyl-4-isoxazolepropionique (AMPA), connu aussi sous le nom de récepteur du quisqualate.

Par ailleurs, les composés de formule (I) sont des antagonistes non compétitifs du récepteur N-méthyl-D-aspartate (NMDA) et, plus particulièrement, ce sont des ligands pour les sites modulateurs de la glycine du récepteur NMDA.

Ces composés sont donc utiles pour traiter ou prévenir toutes les ischémies (telles l'ischémie focale ou globale) consécutives à des accidents vasculaires cérébraux, un arrêt cardiaque, une hypotension artérielle, une intervention chirurgicale cardiaque ou pulmonaire ou une hypoglycémie sévère. Ils sont également utiles dans le traitement des effets dus à une anoxie, qu'elle soit périnatale ou consécutive à une noyade ou à des lésions cérébro-spinales. Ces composés peuvent également être utilisés pour traiter ou prévenir l'évolution de maladies neurodégénératives, de la chorée d'HUNTINGTON, de la maladie d'ALZHEIMER, de la sclérose latérale amyotrophique, de l'atrophie olivo-pontocérébelleuse et de la maladie de PARKINSON. Ces composés peuvent aussi être utilisés vis-à-vis des manifestations épileptogènes et/ou convulsives, pour le traitement des traumatismes cérébraux ou spinaux, des traumatismes liés à la dégénérescence de l'oreille interne (R. PUJOL et coll., Neuroreport, 3, 299-302 (1992) ou de la rétine (J.L. MONSINGER et coll., Exp. Neurol., 113, 10-17 (1991), de l'anxiété (KEHNE et coll., Eur. J. Pharmacol., 193, 283 (1991)), de la dépression (TRULLAS et coll.,Eur. J. Pharmacol., 185, 1 (1990)), de la schizophrénie (REYNOLDS, TIPS, 13, 116 (1992)), du syndrome de TOURETTE, des encéphalopathies hépatiques, en tant qu'analgésiques (DICKENSON et coll., Neurosc. Letters, 121, 263 (1991)), antiinflammatoires (SLUTA et coll., Neurosci. Letters, 149, 99-102 (1993)) antianorexiques (SORRELS et coll., Brain Res., 572, 265 (1992)), antimigraineux, antiémétiques et pour traiter les empoisonnements par des neurotoxines ou d'autres substances agonistes du récepteur NMDA, ainsi que les troubles neurologiques associés aux maladies virales telles que le sida (LIPTON et coll., Neuron, 7, 111 (1991)), la rage, la rougeole et le tétanos (BAGETTA et coll., Br. J. Pharmacol., 101, 776 (1990)). Ces composés sont aussi utiles pour la prévention des symptômes d'abstinence aux drogues et à l'alcool et de l'inhibition de l'accoutumance et de la dépendance aux opiacés. Ils peuvent également être utilisés dans le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, l'hydroxybutirique-aminoacidurie, l'encéphalopathie saturnine (intoxication chronique au plomb) et la déficience en sulfite oxydase.

L'affinité des composés de formule (I) vis-à-vis du récepteur AMPA a été déterminée en étudiant l'antagonisme de la fixation spécifique du [³H]-AMPA sur des membranes de cortex cérébral de rat (HONORE et coll., Neuroscience letters, 54, 27 (1985)). Le [³H]-AMPA est mis à incuber en présence de 0,2 mg de protéines à 4°C pendant 30 minutes dans du tampon KH₂PO₄ 10mM, KSCN 100mM, pH7,5. La fixation non spécifique est déterminée en présence de L-glutamate 1mM. La radioactivité liée est séparée par filtration sur filtres PHARMACIA (Printed Filtermate A). L'activité inhibitrice de ces produits est inférieure ou égale à 100 µM.

L'affinité des composés de formule (I) pour le site glycine lié au récepteur NMDA a été déterminée en étudiant l'antagonisme de la fixation spécifique du [³H]-DCKA sur des membranes de cortex cérébral de rat selon la méthode décrite par T. CANTON et coll., J. Pharm. Pharmacol., 44, 812 (1992). Le [³H]-DCKA (20nM) est mis à incuber en présence de 0,1 mg de protéines à 4°C pendant 30 minutes dans du tampon HEPES 50 mM, pH7,5. La fixation non spécifique est déterminée en présence de glycine 1mM. La radioactivité liée est séparée par filtration sur filtres Whatman GF/B. L'activité inhibitrice de ces produits est inférieure ou égale à 100 µM.

Les composés de formule (I) présentent une toxicité faible. Leur DL50 est supérieure à 50 mg/kg par voie IP chez la souris.

Les composés de formule (I) préférés sont ceux pour lesquels R représente un atome d'hydrogène ou un radical carboxy, alcoxycarbonyle ou carboxamido, R₁ et R₂ sont des atomes d'hydrogène, R₃ et R₄ forment ensemble avec l'atome de carbone auquel ils sont rattachés un cycle 2- ou 3-pyrrolidine, 2- ou 4-pipéridine, ces cycles étant éventuellement substitués sur l'azote par un radical alkyle ou -CO-alk-COOR₆.

Sont d'un intérêt particulier les composés suivants :
- (10'RS)-spiro[pyrrolidine-3,10'-5'H,10'H-imidazo[1,2-a]indéno[1,2-e]pyrazine]-4'-one,
- (10'RS)-spiro[pipéridine-2,10'-5'H,10'H-imidazo[1,2-a]indéno[1,2-e]pyrazine]-4'-one,
- (10'RS)-spiro[pyrrolidine-2,10'-5'H,10'H-imidazo[1,2-a]indéno[1,2-e]pyrazine]-4'-one,
- (10'RS)-1-méthyl-spiro[pyrrolidine-2,10'-5'H,10'H-imidazo[1,2-a]indéno[1,2-e]pyrazine]-4'-one,
- (+)-1 -méthyl-spiro[pyrrolidine-2,10'-5'H,10'H-imidazo[1,2-a]indéno[1,2-e]pyrazine]-4'-one,
- (-)-1 -méthyl-spiro[pyrrolidine-2,10'-5'H,10'H-imidazo[1,2-a]indéno[1,2-e]pyrazine]-4'-one,
- acide (10'RS)-4-oxo-4-(4'-oxo-4',5'-dihydro-spiro[pyrrolidine-2,10'-10'H-imidazo[1,2-a]indéno[1,2-e]pyrazine-1-yl]1-butyrique,
- spiro[pipéridine-4, 10'-5'H, 10'H-imidazo[1,2-a]indéno[1,2-e]pyrazine]-4'-one,

Les exemples suivants illustrent l'invention.

### EXEMPLE 1

A une suspension agitée de 2,35 g de 10-méthylène-5H,10H-imidazo[1,2-a]indéno[1,2-e]pyrazine-4-one dans 150 ml de diméthylformamide on ajoute sous courant d'argon à une température voisine de 20°C 2,79 g de N-n-butoxyméthyl-N-triméthylsilylméthyl-benzylamine en solution dans 25 ml de diméthylformamide et on continue l'agitation jusqu'à dissolution complète. On ajoute alors 0,1 ml d'acide trifluoroacétique et on laisse agiter 3 heures à une température voisine de 20°C, puis 1 heure à 60°C. On élimine ensuite le diméthylformamide au rotavapor et au résidu d'évaporation on ajoute 150 ml de dichlorométhane. La suspension est filtrée et le filtrat est évaporé au rotavapor. Le résidu d'évaporation (3 g) est purifié par chromatographie flash sur silice en éluant avec un mélange dichlorométhane-méthanol (96-4 en volumes). On obtient 1,5 g de (10'RS)-1-benzyl-spiro[pyrrolidine-3,10'-5'H,10'H-imidazo[1,2-a]indéno[1,2-e]pyrazine]-4'-one sous forme de solide blanc (Rf = 0,43, chromatographie sur couche mince de gel de silice, éluant : dichlorométhane-méthanol (9-1 en volumes); Analyse C₂₃H₂₀N₄O % calculé C : 74,98, H : 5,47, N : 15,21, O : 4,34, % trouvé C : 75,0, H : 5,7, N : 15,6, O : 4,8).

La 10-méthylène-5H,10H-imidazo[1,2-a]indéno[1,2-e]pyrazine-4-one peut être préparée de la façon suivante : à une suspension de 19,6 g de 10-hydroxyméthyl-5H,10H-imidazo[1,2-a]indéno[1,2-e]pyrazine-4-one dans 310 ml de méthanol et 39 ml de diméthylsulfoxyde on ajoute sous agitation et sous atmosphère d'argon 155 ml d'une solution normale de soude aqueuse et on continue l'agitation pendant 16 heures à une température voisine de 20°C. Le milieu réactionnel est alors acidifié avec 194 ml d'une solution normale d'acide chlorhydrique aqueux et le précipité qui apparaît est filtré, lavé à l'eau distillée (2x50 ml), puis à l'acétone (2x50 ml) et séché d'abord à l'air et enfin sous vide (2 mm Hg; 0,26 kPa) à 60°C. On obtient ainsi 14 g de 10-méthylène-5H,10H-imidazo[1,2-a]indéno[1,2-e]pyrazine-4-one sous forme de solide jaune (Rf = 0,53, chromatographie sur couche mince de gel de silice, éluant : chloroforme-méthanol (8-2 en volumes)).

Le 10-hydroxyméthyl-5H,10H-im idazo[1,2-a]indéno[1,2-e]pyrazine-4-one peut être préparé de la manière suivante : à une suspension de 3 g de 10-hydroxyméthylène-5H,10H-imidazo[1,2-a]indéno[1,2-e]pyrazine-4-one (mélange des formes Z et E) dans 240 ml de méthanol, on ajoute sous agitation en 10 minutes et par portions 1 g de borohydrure de sodium, et on poursuit l'agitation à une température voisine de 20°C pendant 1 heure et 30 minutes. On ajoute à nouveau 1 g de borohydrure de sodium par portions et on maintient l'agitation pendant 30 minutes. Le mélange réactionnel est alors filtré et le filtre est rincé avec 2 fois 30 ml de méthanol. L'insoluble est repris avec 50 ml d'eau et 4 ml d'acide chlorhydrique 1N, filtré à nouveau, lavé à l'eau jusqu'à neutralité et séché à l'air. Le produit brut (1g) est purifié par dissolution à chaud dans 65 ml de diméthylformamide, filtration, addition au filtrat encore chaud de 80 ml de méthanol et cristallisation dans un bain d'eau et de glace. Les cristaux obtenus sont filtrés, lavés avec 2 fois 15 ml de méthanol et séchés à 80°C. On obtient ainsi 0,66 g de 10-hydroxyméthyl-5H,10H-imidazo[1,2-a]indéno[1,2-e]pyrazine-4-one sous forme de solide blanc fondant au-dessus de 260°C (Analyse % calculé C : 66,40, H : 4,38, N : 16,59, O : 12,63, % trouvé C : 66,4, H : 4,3, N : 16,6, O : 12,1).

Le 10-hydroxyméthylène-5H,10H-imidazo[1,2-a]indéno[1,2-e]pyrazine-4-one peut être préparé de la manière suivante : une solution de 1,4 g de 10-(E)-diméthylaminométhylène-5H,10H-imidazo[1,2-a]indéno[1,2-e]pyrazine-4-one dans 35 ml d'acide chlorhydrique 5N est agitée pendant 30 minutes à une température voisine de 25°C. Après addition de 60 ml d'eau distillée et neutralisation par 120 ml d'une solution aqueuse saturée d'hydrogénocarbonate de sodium, le solide apparu est séparé par filtration, lavé 2 fois par 60 ml au total d'eau distillée et séché à l'air. Le produit obtenu (1,1 g) est dissous dans 120 ml de diméthylsulfoxyde et, après addtion de 120 ml d'eau distillée, le solide apparu est séparé par filtration, lavé 2 fois avec 10 ml au total d'eau distillée et 2 fois avec 10 ml au total d'acétone puis séché sous pression réduite (1 mm Hg; 0,13 kPa) à 100°C. On obtient ainsi 1 g de 10-hydroxyméthylène-5H,10H-imidazo[1,2-a]indéno[1,2-e]pyrazine-4-one, mélange 60-40 des formes Z et E, se décomposant sans fondre à 290°C [Spectre de R.M.N. : (200 MHz; DMSO d6; δ en ppm) : on observe un mélange d'isomères 60/40 : de 7,20 à 7,40 (mt, 2H : -H7 et -H8); 7,56 et 7,64 - 8,29 et 8,79 (4s larges, 2fois 1H : -H de l'imidazole); de 7,80 à 8,15 (mt, 2H :-H6 et -H9); 8,21 et 8,24 (2s, 1H en totalité : =CH-O-); 12,43 (mf, 1H: -NH-)].

Le 10-(E)-diméthylam inométhylène-5H,10H-imidazo[1,2-a]indéno[1,2-e]pyrazine-4-one peut être préparé de la manière suivante : 6,3 g de t-butoxy-bis(diméthylamino)méthane sont ajoutés goutte à goutte à une température voisine de 25°C en 5 minutes à une suspension de 5,5 g de 5H,10H-imidazo[1,2-a]indéno[1,2-e]pyrazine-4-one dans 100 ml de diméthylformamide. Après 30 minutes d'agitation à la même température, le mélange est versé sur 500 ml d'eau distillée et extrait 5 fois avec 1,5 litre au total de chloroforme. Les extraits organiques sont réunis, lavés avec 250 ml d'eau distillée, séchés sur du sulfate de magnésium anhydre et concentrés à sec sous pression réduite (15 mm Hg; 2 kPa) à 60°C. Le produit obtenu (4,5 g) est mis en suspension dans 25 ml de méthanol, filtré, lavé 2 fois avec 20 ml au total de méthanol et séché sous pression réduite (15 mm Hg; 2 kPa) à 20°C. Le produit obtenu (4,5 g) est dissous dans 45 ml de diméthylformamide bouillant et la solution, après refroidissement, est conservée pendant 4 heures à une température voisine de 5°C. Les cristaux sont séparés par filtration, lavés successivement avec 10 ml de diméthylformamide, 10 ml d'acétone et séchés à sec sous pression réduite (1 mm Hg; 0,13 kPa) à 100°C. On obtient ainsi 4 g de 10-(E)-diméthylaminométhylène-5H,10H-imidazo[1,2-a]indéno [1,2-e]pyrazine-4-one fondant à 293°C [Spectre de R.M.N. : (200 MHz; DMSO d6; δ en ppm) : 3,35 [s, 6H : -N(CH3)2]; 7,18 et 7,28 (2t, J = 7,5 Hz, 2H : -H7 et -H8); 7,48 et 7,92 (2d, J = 7,5 Hz, 1H chacun : -H6 et -H9); 7,63 et 8,50 (2s larges, 1H chacun : -H de l'imidazole); 8,09 (s, 1H : =CH-N); 12,30 (mf, 1H: -NH-)].

La 5H,10H-imidazo[1,2-a]indéno[1,2-e]pyrazine-4-one peut être préparée de la manière suivante : une solution de 4,8 g de bromure de 3-méthyl-4-oxo-5H,10H-imidazo[1,2-a]indéno[1,2-e]pyrazinium dans 30 g d'imidazole est chauffée pendant 24 heures à 160°C, refroidie à 100°C puis versée sur un mélange agité de 75 g de glace et de 75 g d'eau distillée. L'insoluble est filtré, lavé 2 fois avec 20 ml au total d'eau distillée puis séché sous pression réduite (10 mm Hg; 1,3 kPa) à 50°C. Le produit ainsi obtenu (4 g) est dissous dans 80 ml de diméthylformamide et la solution addtionnée de 20 g de silice est concentrée à sec sous pression réduite (15 mm Hg, 2 kPa) à 100°C. Le mélange est introduit dans une colonne de 4,2 cm de diamètre contenant 240 g de silice puis est élué par un mélange dichlorométhane-méthanol (97-3 en volumes) en recueillant des fractions de 60 ml . Les fractions 10 à 70 sont réunies, additionnées de 1,5 g de noir décolorant, filtrées et concentrées à sec sous pression réduite (15 mm Hg; 2 kPa) à 55°C. Le produit obtenu (1,7 g) est dissous dans 350 ml de méthanol bouillant et la solution additionnée de 0,1 g de noir décolorant est filtrée à chaud, concentrée sous pression réduite (15 mm Hg; 2 kPa) à 40°C pour ramener son volume à environ 30 ml puis conservée à 5°C pendant 60 heures. Les cristaux sont séparés par filtration, lavés 2 fois avec 20 ml au total de méthanol glacé et séchés sous pression réduite (1 mm Hg; 0,13 kPa) à 60°C. On obtient ainsi 1,1 g de 5H,10H-imidazo[1,2-a]indéno[1,2-e]pyrazine-4-one se décomposant sans fondre à 350°C [Rf= 0,77, chromatographie sur couche mince de gel de silice, solvant : dichlorométhane-méthanol (8-2 en volumes)].

Le bromure de 3-méthyl-4-oxo-5H,10H-imidazo[1,2-a]indéno[1,2-e]pyrazinium peut être préparé de la manière suivante : une solution de 5 g de 1-méthyl-1H-imidazole-2-carboxamide et de 12 g de 2-bromoindanone à 85% dans 100 ml de diméthylformamide anhydre est agitée pendant 28 heures à 115°C puis refroidie à une température voisine de 20°C. L'insoluble est séparé par filtration, lavé 2 fois avec 20 ml au total de diméthylformamide glacé et séché sous pression réduite (10 mm Hg; 1,3 kPa). On obtient ainsi 4,8 g de bromure de 3-méthyl-4-oxo-5H,10H-imidazo[1,2-a]indéno[1,2-e]pyrazinium [Spectre de R.M.N.: (200 MHz; DMSO d6; δ en ppm) : 4,13 (s, 2H : -CH2 en 10); 4,34 (s, 3H : N+ -CH3); 7,47 (mt, 2H : -H7 et -H8); 7,68 et 7,96 (2d, J = 7,5 Hz, 1H chacun : -H6 et -H9); 8,32 et 8,45 (2d, J = 1 Hz, 1H chacun : H de l'imidazole); 13,60 (mf, 1H : NH)].

Le 1-méthyl-1H-imidazole-2-carboxamide peut être préparée selon le procédé décrit par D.D. DAVEY, J. Org. Chem., 52, 4379 (1987).

La N-n-butoxyméthyl-N-triméthylsilylméthyl benzylamine peut être préparée selon la méthode décrite par Y. TERAO et coll., Chem. Pharm. Bull., 33, 2762 (1985).

### EXEMPLE 2

On hydrogène à une température voisine de 60°C en autoclave un mélange de 1 g de (10'RS)-1-benzyl-spiro[pyrrolidine-3,10'-5'H,10'H-imidazo[1,2-a]indéno[1,2-e]pyrazine]-4'-one, 50 ml d'acide acétique et 0,2 g d'hydroxyde de palladium sur charbon à 20% sous une pression de 10 bar pendant 3 heures. Après élimination du catalyseur sous atmosphère inerte, le mélange réactionnel est évaporé au rotavapor et le résidu d'évaporation est dissous dans l'eau. La solution aqueuse est neutralisée avec une solution d'hydrogénocarbonate de sodium pour donner un précipité que l'on filtre, lave à l'eau distillée et sèche à 35°C sous vide (1 mm Hg; 0,13 kPa). On obtient 0,25 g de (10'RS)-spiro[pyrrolidine-3,10'-5'H,10'H-imidazo[1,2-a]indéno[1,2-e]pyrazine]-4'-one sous forme de solide de couleur crème fondant au-dessus de 260°C (Analyse % calculé C : 69,05, H : 5,07, N : 20,13, O : 5,75, % trouvé C : 69,2, H : 5,0, N : 19,7, O : 5,0).

### EXEMPLE 3

A une suspension agitée de 1,7 g de 10-tert-butoxycarbonylamino-5H,10H-imidazo[1,2-a]indéno[1,2-e]pyrazine-4-one dans 15 ml de diméthylsulfoxyde on ajoute par petites quantités 0,6 g d'hydrure de sodium à 80% et on continue l'agitation pendant 15 minutes. A la solution brune obtenue on ajoute en 3 minutes 0,63 ml de 1-bromo-4-chlorobutane en solution dans 1,5 ml de diméthylformamide et on laisse agiter pendant 2 heures et 30 minutes à une température voisine de 20°C. Le milieu réactionnel est versé sur un mélange de 50 g de glace pilée, 100 ml d'eau distillée et 2 ml d'acide acétique. La suspension obtenue est filtrée et l'insoluble est lavé à l'eau distillée (2x20 ml), puis avec 20 ml d'acétone et séché à l'air pendant une nuit. On obtient 1,37 g de (10'RS)-1-tert-butoxycarbonyl-spiro[pipéridine-2,10'-5'H,10'H-imidazo[1,2-a]indéno[1,2-e]pyrazine]-4'-one sous forme de solide de couleur crème fondant au-dessus de 260°C (Rf = 0,86, chromatographie sur couche mince de gel de silice, éluant : chloroforme-méthanol-ammoniaque à 28% (12-3-0,5 en volumes)). La déprotection du NH s'effectue de la manière suivante : à 11 ml d'acide trifluoroacétique on ajoute sous agitation et par portions 1,36 g de (10'RS)-1-tert-butoxycarbonyl-spiro[pipéridine-2,10'-5'H,10'H-imidazo[1,2-a]indéno[1,2-e]pyrazine]-4'-one et on poursuit l'agitation à une température voisine de 20°C pendant 2 heures et 30 minutes. Le milieu réactionnel est évaporé au rotavapor et le résidu d'évaporation est traité avec 35 ml d'eau distillée et neutralisé à pH 7 en ajoutant de l'hydrogénocarbonate de sodium par petites portions. La suspension obtenue est filtrée et l'insoluble est lavé à l'eau distillée (3x20 ml), puis avec 20 ml d'isopropanol. Après séchage à 60°C sous vide (1 mm Hg; 0,13 kPa), on obtient 0,9 g de (10'RS)-spiro[pipéridine-2,10'-5'H,10'H-imidazo[1,2-a]indéno[1,2-e]pyrazine]-4'-one sous forme de solide rose pâle fondant à 268°C en se décomposant (Analyse % calculé C : 69,85, H : 5,52, N : 19,16, O : 5,47, % trouvé C : 69,8, H : 5,8, N : 19,4).

La 10-tert-butoxycarbonylamino-5H,10H-imidazo[1,2-a]indéno[1,2-e]pyrazine -4-one peut être préparée de la manière suivante : à une suspension agitée de 39,2 g de chlorhydrate de 10-amino-5H,10H-imidazo[1,2-a]indéno[1,2-e] pyrazine-4-one dans 400 ml de diméthylformamide on ajoute sous courant d'azote à une température voisine de 30°C 22,4 g de dicarbonate de di-tert-butyle, puis une solution de 25 ml de triéthylamine dans 35 ml de diméthylformamide, et on poursuit l'agitation pendant 20 heures. Le mélange réactionnel est filtré et l'insoluble est lavé avec 50 ml de diméthylformamide, puis avec de l'eau distillée (3x100 ml) et enfin avec 100 ml d'acétone. Après séchage à l'air, puis sous vide (1 mm Hg; 0,13 kPa) à 100°C on obtient 30,6 g de 10-tert-butoxycarbonylamino-5H,10H-imidazo[1,2-a]indéno[1,2-e]pyrazine-4-one sous forme de solide jaune pâle fondant au-dessus de 260°C (Rf = 0,64, chromatographie sur couche mince de gel de silice, éluant : chloroforme-méthanol-ammoniaque à 28% (12-3-0,5 en volumes)).

La 10-amino-5H,10H-imidazo[1,2-a]indéno[1,2-e]pyrazine-4-one peut être préparée de la manière suivante : une solution de 12,9 g de 10-acétamido-5H,10H-imidazo[1,2-a]indéno[1,2-e]pyrazine-4-one dans 650 ml d'une solution aqueuse 2N d'acide chlorhydrique est chauffée à l'ébullition pendant 2 heures, refroidie puis concentrée à sec sous pression réduite (15 mm Hg; 2 kPa) à 80°C. 4 g (sur les 14,8 g obtenus au total) sont dissous dans 250 ml d'eau distillée et la solution est agitée pendant 16 heures à une température voisine de 20°C. Les cristaux apparus sont séparés par filtration, lavés successivement avec 25 ml d'eau distillée et 25 ml de méthanol puis séchés à l'air à une température voisine de 20°C. Le produit obtenu (3,5 g) est agité en suspension pendant 10 minutes dans 100 ml de méthanol bouillant et, après refroidissement et conservation pendant 1 heure à 5°C, isolé par filtration, lavé avec 20 ml de méthanol glacé puis séché sous pression réduite (1 mm Hg; 0,13 kPa) à 100°C. On obtient ainsi 2,1 g de chlorhydrate de 10-amino-5H,10H-imidazo[1,2-a]indéno[1,2-e]pyrazine-4-one se décomposant sans fondre vers 240°C [Spectre de R.M.N. : (200 MHz; DMSO d6; δ en ppm) : 5,70 (s large, 1H : CH-N+-Cl-); 7,48 et 7,58 (2t, J = 7,5 Hz, 1H chacun: -H7 et -H8); 7,72 et 8,76 (2s, 1H chacun : -H de l'imidazole); 7,98 et 8,09 (2d, J = 7,5 Hz, 1H chacun : -H6 et -H9); 9,47 (mf, 3H : N+H3Cl-); 12,80 (mf, 1H: -NH-)].

La 10-acétamido-5H,10H-imidazo[1,2-a]indéno[1,2-e]pyrazine-4-one peut être préparée de la manière suivante : une suspension de 5,25 g de 10-(E-hydroxyimino)-5H,10H-imidazo[1,2-a]indéno[1,2-e]pyrazine-4-one et de 2,9 g de zinc en poudre dans 100 ml d'acide acétique est chauffée pendant 2 heures à une température comprise entre 80°C et 90°C. Après addition de 100 ml d'acide acétique, le mélange est filtré et le filtrat est concentré à sec sous pression réduite (10 mm Hg; 2 kPa) à 65°C. Le produit obtenu (3,8 g) est mis en suspension dans 100 ml d'eau distillée, filtré, lavé avec 10 ml d'eau distillée et avec 10 ml d'acétone puis séché sous pression réduite (15 mm Hg; 2 kPa) à 20°C. Le produit obtenu (2 g) est dissous dans 60 ml de diméthylformamide bouillant et la solution, additionnée de 0,1 g de noir décolorant est filtrée à chaud. Le filtre est lavé avec 10 ml de diméthylformamide bouillant puis le filtrat et le lavage réunis sont conservés pendant 4 heures à une température voisine de 20°C. Les cristaux apparus sont séparés par filtration, lavés successivement avec 10 ml de diméthylformamide, 10 ml d'eau distillée, 10 ml d'acétone et séchés à sec sous pression réduite (1mm Hg; 0,13 kPa) à 100°C. On obtient ainsi 0,43 g de 10-acétamido-5H,10H-imidazo[1,2-a]indéno[1,2-e]pyrazine-4-one se décomposant sans fondre à 330°C [Spectre de R.M.N. : (200 MHz; DMSO d6; δ en ppm) : 2,00 (s, 3H : -CO-CH₃); 6,13 (d, J = 8,5 Hz, 1H : CH-N); 7,35 et 7,48 (2t, J = 7,5 Hz, 1H chacun : -H7 et -H8); 7,48 et 7,85 (2d, J = 7,5 Hz, 1H chacun : -H6 et -H9); 7,58 et 7,65 (2s larges, 1H chacun : -H de l'imidazole); 8,58 (d, J = 8,5 Hz, 1H: -NH-COCH₃); 12,50 (mf, 1H: -NH-)].

La 10-(E-hydroxyimino)-5H,10H-imidazo[1,2-a]indéno[1,2-e]pyrazine-4-one peut être préparée de la manière suivante : 0,4 g d'hydrure de sodium à 80% est ajouté à une suspension de 1,1 g de 5H,10H-imidazo[1,2-a]indéno[1,2-e]pyrazine-4-one dans 10 ml de diméthylsulfoxyde anhydre. Après 10 minutes d'agitation à une température voisine de 20°C, une solution de 0,7 g de nitrite d'isoamyle dans 2 ml de diméthylsulfoxyde anhydre est ajoutée goutte à goutte en 5 minutes puis le mélange est agité pendant 1 heure à la même température. 10 ml d'eau distillée sont ajoutés lentement et le mélange est ensuite versé sur 120 g d'eau et de glace, acidifié avec 1 ml d'acide acétique puis centrifugé. Après élimination de la solution surnageante, le solide est mis en suspension dans 25 ml d'eau distillée, filtré, lavé avec 10 ml d'acétone et séché sous pression réduite (15 mm Hg; 2 kPa) à 20°C. Le produit obtenu (1,5 g) est dissous dans 100 ml de diméthylformamide bouillant et la solution, additionnée de 0,1 g de noir décolorant, est filtrée à chaud, refroidie, versée sur 800 ml d'eau distillée et centrifugée. Le solide est mis en suspension dans 20 ml d'eau distillée, filtré, lavé avec 20 ml d'acétone et séché sous pression réduite (15 mm Hg; 2 kPa) à 20°C. Le produit obtenu (0,9 g) est dissous dans 75 ml de diméthysulfoxyde à 20°C et la solution, additionnée de 0,1 g de noir décolorant est filtrée. Le filtre est lavé 2 fois avec 20 ml au total de diméthylsulfoxyde puis le filtrat et le lavage sont réunis, additionnés de 75 ml d'eau distillée et centrifugés. Le solide est mis en suspension dans 25 ml d'eau distillée, filtré, lavé 2 fois avec 50 ml au total d'acétone puis séché sous pression réduite (1 mm Hg; 0,13 kPa) à 60°C. On obtient ainsi 0,63 g de 10-(E-hydroxyimino)-5H,10H-imidazo[1,2-a]indéno[1,2-e]pyrazine-4-one se décomposant sans fondre au-dessus de 300°C [Spectre de R.M.N.: (200 MHz; DMSO d6; δ en ppm) : 7,40 et 7,48 (2t, J = 7 Hz, 2H: -H7 et -H8); 7,60 et 8,00 (2s larges, 1H chacun : -H de l'imidazole); 7,82 et 8,20 (2d, J = 7 Hz, 1H chacun : -H6 et -H9); 12,70 et 13,00 (2mf, 1H chacun : -NH- et -OH)].

### EXEMPLE 4

On opère comme à l'exemple 3 mais à partir de 0,7 g de 10-tert-butoxycarbonylamino-5H,10H-imidazo[1,2-a]indéno[1,2-e]pyrazine-4-one, 20,5 ml de diméthylsulfoxyde, 0,24 g d'hydrure de sodium à 80% et 0,22 ml de 1-bromo-3-chloropropane. Après hydrolyse, le milieu réactionnel est soumis à des extractions à l'acétate d'éthyle (4x200 ml). Les phases organiques sont réunies; elles contiennent un insoluble qui est filtré, lavé avec 10 ml d'eau distillée, puis avec 10 ml d'acétate d'éthyle et séché à l'air pour donner 0,29 g de produit brut (A). Les phases organiques sont réunies sont lavées à l'eau distillée (150 ml) et évaporées au rotavapor. Le résidu d'évaporation (0,41 g) est trituré avec 5 ml d'acétone, filtré, lavé avec 2 ml d'acétone et séché à l'air pour donner 0,18 g de produit brut (B). Les produits bruts (A) et (B) sont réunis et purifiés par chromatographie flash sur silice en éluant avec un mélange chloroforme-méthanol-ammoniaque à 28% (12-2,25-0,38 en volumes). On obtient 0,43 g de (10'RS)-1-tert-butoxycarbonyl-spiro[pyrrolidine-2,10'-5'H,10'H-imidazo[1,2-a]indéno[1,2-e]pyrazine]-4'-one sous forme de solide de couleur crème fondant au-dessus de 260°C (Analyse C₂₁H₂₂N₄O₃ % calculé C : 66,65, H : 5,86, N : 14,81, O : 12,68, % trouvé C : 67,0, H : 5,1, N : 14,5).

### EXEMPLE 5

A 6 ml d'acide trifluoroacétique on ajoute sous agitation et par portions 0,42 g de (10'RS)-1-tert-butoxycarbonyl-spiro[pyrrolidine-2,10'-5'H,10'H-imidazo[1,2-a]indéno[1,2-e]pyrazine]-4'-one et on poursuit l'agitation à une température voisine de 20°C pendant 2 heures et 30 minutes. Le milieu réactionnel est évaporé au rotavapor et le résidu d'évaporation est traité avec 15 ml d'eau distillée et neutralisé à pH 7 en ajoutant de l'hydrogénocarbonate de sodium par petites portions. La suspension obtenue est filtrée et l'insoluble est lavé à l'eau distillée (3x20 ml), puis avec 20 ml d'isopropanol. Après séchage à 60°C sous vide (1 mm Hg; 0,13 kPa), on obtient 0,15 g de (10'RS)-spiro[pyrrolidine-2,10'-5'H,10'H-imidazo[1,2-a]indéno[1,2-e]pyrazine]-4'-one sous forme de solide de couleur crème se décomposant vers 260°C au bloc Maquenne (Analyse % calculé C : 69,05, H : 5,07, N : 20,13, O : 5,75, % trouvé C : 69,4, H : 5,0, N : 19,7).

### EXEMPLE 6

Pendant 1 heure on agite à une température voisine de 28°C un mélange de 3 ml d'une solution de formaldéhyde à 37%, 1,1 g de (10'RS)-spiro[pyrrolidine-2, 10'-5'H,10'H-imidazo[1,2-a]indéno[1,2-e]pyrazine]-4'-one et 1,5 ml d'acide formique. La solution obtenue est ensuite évaporée au rotavapor. Le résidu d'évaporation est trituré avec 60 ml d'eau distillée, filtré, lavé à l'eau distillée (4x5 ml) et séché à l'air. Après séchage à 60°C sous vide (1 mmHg; 0,13 kPa) on obtient 0,62 g de (10'RS)-1-méthyl-spiro[pyrrolidine-2,10'-5'H,10'H-imidazo[1,2-a]indéno[1,2-e]pyrazine]-4'-one sous forme de solide de couleur crème fondant à 230°C avec décomposition au bloc Maquenne [Spectre de R.M.N. 1H (200 MHz, (CD₃)₂SO d6 avec ajout de quelques gouttes de CD_{3,}COOD d4, δ en ppm) : de 1,70 à 2,10 et de 2,20 à 2,70 (2 mts, 2H chacun : CH₂CH₂); 1,82 (s, 3H : NCH₃); de 3,00 à 3,50 (mt, 2H : NCH₂); de 7,20 à 7,50 et 7,85 (respectivement mt et d (J = 7,5 Hz), 3H et 1H : H Aromatique); 7,60 et 7,90 (2s, 1H chacun: H de l'imidazole)].

### EXEMPLE 7

Par chromatographie de 4 fois 0,8 g de (+/-)-1-méthyl-spiro[pyrrolidine-2,10'-5'H,10'H-imidazo[1,2-a]indéno[1,2-e]pyrazine]-4'-one sur colonne chirale type CHIRACEL OC (longueur=250 mm; diamètre=60 mm) en utilisant comme éluant de l'éthanol absolu avec un débit de 30 ml/minute, on obtient 1,40 g de (+)-1 -méthyl-spiro[pyrrolidine-2,10'-5'H,10'H-imidazo[1,2-a]indéno[1,2-e]pyrazine]-4'-one sous forme de solide de couleur crème [α_{D}²⁰ = +32,4 (acide acétique; c=0,5%)] et 1,36 g de (-)-1-méthyl-spiro[pyrrolidine-2,10'-5'H,10'H-imidazo[1,2-a]indéno[1,2-e]pyrazine]-4'-one sous forme de solide de couleur crème [α_{D}²⁰ = -32,0 (acide acétique; c=0,5%)].

### EXEMPLE 8

A une solution agitée de 0,7 g de (10'RS)-spiro[pyrrolidine-2,10'-5'H,10'H-imidazo[1,2-a]indéno[1,2-e]pyrazine]-4'-one dans 8 ml d'acide acétique on ajoute 0,6 g d'anhydride succinique et on poursuit l'agitation pendant 48 heures à une température voisine de 20°C. La suspension obtenue est filtrée et l'insoluble est lavé avec 3 ml d'acide acétique, puis à l'eau distillée (3x3 ml) et enfin avec 3 ml d'acétone. Après séchage à 60°C sous vide (1 mm Hg; 0,13 kPa) on obtient 0,28 g d'acide (10'RS)-4-oxo-4-(4'-oxo-4',5'-dihydro-spiro[pyrrolidine-2,10'-10'H-imidazo[1,2-a]indéno[1,2-e]pyrazine-1-yl])-butyrique sous forme de solide de couleur crème fondant à 300°C avec décomposition au bloc Maquenne [Spectre de R.M.N. ¹H (200 MHz, (CD₃)₂SO d6 avec ajout de quelques gouttes de CD_{3,}COOD d4, à une température de 373 K, δ en ppm) : de 1,80 à 2,70 (mts, 8H : CH₂); 4,15 (mt, 2H : NCH₂); de 7,20 à 7,60 et 7,85 (respectivement mt et d (J = 7,5 Hz), 3H et 1H : H Aromatique); 7,55 et 7,59 (2s, 1H chacun : H de l'imidazole)].

### EXEMPLE 9

A une suspension de 0,89 g de 5H,10H-imidazo[1,2-a]indé no[1,2-e]pyrazine-4-one dans 10 ml de diméthylsulfoxyde, sous azote, on ajoute une solution de 1,18 g de N,N-bis(2-chloroéthyl)-p-toluènesulfonamide dans 5 ml de diméthylsulfoxyde puis progressivement et sous forte agitation 0,8 g d'hydrure de sodium à 60% en environ 30 minutes. L'agitation est poursuivie 20 heures à une température voisine de 20°C. Après addition de méthanol puis d'eau, le mélange est versé sur de la glace et acidifié à l'aide d'acide chlorhydrique 1N. L'insoluble est filtré, lavé avec de l'éthanol absolu et séché sous pression réduite (1 mm Hg; 0,13 kPa) à 50°C pour conduire à 0,9 g de 1-tosyl-spiro[pipéridine-4,10'-5'H,10'H-imidazo[1,2-a]indéno[1,2-e] pyrazine]-4'-one dont le point de fusion est supérieur à 260°C [Spectre de R.M.N. ¹H (400 MHz, (CD₃)₂SO d6 avec ajout de quelques gouttes de CD₃COOD d4, à une température de 333 K, δ en ppm) : 1,42 (d large, J = 14 Hz, 2H : H équatorial des CH₂ de la pipéridine); 2,50 (mt, 2H : H axial des CH₂ de la pipéridine); 2,52 (s, 3H : ArCH₃); 3,27 (t large, J = 14 Hz, 2H : H axial des NCH₂ de la pipéridine); 3,97 (d large, J = 14 Hz, 2H : H équatorial des NCH₂ de la pipéridine); de 7,20 à 8,00 (mt, 10H : H Aromatiques et H de l'imidazole)] dont le NH est ensuite déprotégé de la manière suivante : une suspension de 0,9 g de 1-tosyl-spiro[pipéridine-4,10'-5'H,10'H-imidazo [1,2-a]indéno[1,2-e]pyrazine]-4'-one dans 20 ml d'acide bromhydrique à 47% est portée à reflux pendant 5 heures. Après refroidissement à une température voisine de 20°C et addition d'environ 20 ml d'eau distillée, le milieu réactionnel est neutralisé à l'aide de soude aqueuse concentrée. Le précipité est filtré, repris dans l'acide chlorhydrique 1N, l'insoluble est écarté et le filtrat neutralisé à l'aide de soude aqueuse concentrée. Le précipité est filtré, lavé à l'eau et séché sous pression réduite (1 mmHg; 0,13 kPa) à 50°C. On obtient ainsi 0,1 g de chlorhydrate de spiro[pipéridine-4,10'-5'H,10'H-imidazo[1,2-a] indéno[1,2-e]pyrazine]-4'-one, sous forme de poudre verte dont le point de fusion est supérieur à 260°C [Spectre de R.M.N. ¹H (400 MHz, (CD₃)₂SO d6 avec ajout de quelques gouttes de CD_{3,}COOD d4, δ en ppm) : 1,55 (d large, J = 14 Hz, 2H : H équatorial des CH₂ de la pipéridine); 2,92 (dt, J = 14 et 5,5 Hz, 2H : H axial des CH₂ de la pipéridine); de 3,50 à 3,75 (mt 4H : NCH₂ de la pipéridine); de 7,30 à 8,05 (mt, 4H : H Aromatiques); 7,62 et 8,65 (2 s, 1H chacun : H de l'imidazole)].

### EXEMPLE 10

On chauffe à reflux pendant 2 heures un mélange de 373 mg de bromhydrate de spiro[pipéridine-4,10'-5'H,10'H-imidazo[1,2-a]indéno[1,2-e]pyrazine]-4'-one, 20 ml de dioxane, 0,14 ml de triéthylamine, 110 mg d'anhydride succinique et 135 mg de 4-diméthylaminopyridine. Le mélange réactionnel est flitré et le solide obtenu est lavé à l'acide chlorhydrique 1N, puis à l'eau (4x5 ml) et enfin à l'éther éthylique. Après séchage à 35°C sous vide (1 mmHg; 0,13 kPa) on obtient 180 mg d'acide 4-oxo-4-(4'-oxo-4',5'-dihydro-spiro[pipéridine-4,10'-10'H-imidazo[1,2-a]indéno[1,2-e]pyrazine-1-yl])butyrique sous forme de solide grisâtre fondant au-dessus de 260°C (Analyse C₂₁H₂₀N₄O₄ % calculé C : 64,28, H : 5,14, N : 14,28, O : 16,31, % trouvé C : 64,3, H : 5,2, N : 14,3).

### EXEMPLE 11

A un mélange agité à une température voisine de 20°C de 730 mg de spiro[pipéridine-4, 0'-5'H, 10'H-imidazo[1,2-a]indéno[1,2-e]pyrazine]-4'-one et de 15 ml de diméthylformamide on ajoute goutte à goutte 0,42 ml de triéthylamine. On refroidit le milieu réactionnel vers 5°C et on ajoute goutte à goutte une solution de 0,21 ml de chlorure d'acétyle dans 10 ml de diméthylformamide. On poursuit l'agitation durant la nuit en laissant la température de mélange réactionnel remonter aux environs de 20°C et on ajoute 15 ml d'eau. Le milieu réactionnel est soumis à 4 extractions au dichlorométhane (140 ml au total) et la phase organique est lavée à l'eau distillée (3x20 ml), séchée sur sulfate de magnésium, filtrée et évaporée au rotavapor. On obtient 100 mg de 1-acétyl-spiro[pipéridine-4,10'-5'H,10'H-imidazo[1,2-a]indéno[1,2-e]pyrazine]-4'-one sous forme de solide jaune fondant au-dessus de 260°C [Spectre de R.M.N.: (250 MHz; DMSO d6; δ en ppm) : 1,35 et 1,42 (1H, d, J = 12 Hz, CH éq.), 2,18 (3H, s, COCH3), 2,30 et 2,55 (1H, m, CH ax.), 3,28 et 3,82 (1H, t, J = 12 Hz, NCH ax.), 4,04 et 4,65 (1H, d, J = 12 Hz, NCH éq.), 7,35 (1H, t, J = 7 Hz, CH arom.), 7,48 (1H, t, J = 7 Hz, CH arom.), 7,62 (1H, s, CH arom.), 7,97 (1H, d, J = 7 Hz, CH arom.), 8,09 (1H, d, J = 7 Hz, CH arom.), 8,15 (1H, s, CH arom.), 12,42 (1H, s, NH)].

### EXEMPLE 12

On opère comme à l'exemple 11 mais à partir de 746 mg de bromhydrate de spiro[pipéridine-4,10'-5'H,10'H-imidazo[1 ,2-a]indéno[1,2-e]pyrazine]-4'-one, 0,62 ml de triéthylamine et 0,29 ml de chlorure de phénylacétyle. Le résidu d'évaporation se présente comme une huile brune. Après trituration dans l'acétonitrile cette huile fournit un précipité qui est filtré, lavé à l'acétonitrile et séché à 40°C sous vide (1 mmHg; 0,13 kPa). On obtient ainsi 390 mg de 1-phénylacétyl-spiro[pipéridine-4,10'-5'H,10'H-imidazo[1,2-a]indéno[1,2-e]pyrazine]-4'-one sous forme de solide gris clair fondant au-dessus de 260°C (Analyse C₂₅H₂₂N₄O₂ % calculé C : 73,15, H : 5,40, N : 13,65, O : 7,80, % trouvé C : 72,8, H : 5,5, N : 14,0, O: 7,7).

### EXEMPLE 13

On opère comme à l'exemple 11 mais à partir de 1 g de bromhydrate de spiro[pipéridine-4,1 O'-5'H,10'H-imidazo[1,2-a]indéno[1,2-e]pyrazine]-4'-one, 40 ml de diméthylformamide, 0,75 ml de triéthylamine et 0,44 ml de chlorure d'hydrocinnamoyle. On obtient 170 mg de 1-(3-phénylpropionyl)-spiro[pipéridine-4,10'-5'H,10'H-imidazo[1,2-a]indéno[1,2-e]pyrazine]-4'-one sous forme de solide beige fondant vers 232°C avec décomposition [Spectre de R.M.N.: (200 MHz; DMSO d6 + CD₃CO₂D; δ en ppm) : 1,40 (1H, d, J = 14 Hz, CH éq.), entre 2,5 et 3,00 (5H, m, CH ax., et 2 CH₂), 3,30 et 3,75 (1H, t, J = 14 Hz, NCH ax.), 4,10 et 4,70 (1H, d, J = 14 Hz, NCH éq.), 7,23 (1H, t, J = 7 Hz, CH arom.), entre 7,25 et 7,40 (5H, m, H phényl), 7,45 (1H, t, J = 7 Hz, CH arom.), 7,61 (1H, s, CH arom.), 7,95 (1H, s, CH arom.), 7,97 (1H, d, J = 7 Hz, CH arom.), 8,03 (1H,d, J = 7 Hz, CH arom.)].

### EXEMPLE 14

On opère comme à l'exemple 11 mais à partir de 1,1 g de bromhydrate de spiro[pipéridine-4,10'-5'H,10'H-imidazo[1,2-a]indéno[1,2-e]pyrazine]-4'-one, 40 ml de diméthylformamide, 1,25 ml de triéthylamine et 600 mg de chlorure de 3-diéthylaminopropionyle, chlorhydrate. Le milieu réactionnel est concentré au rotavapor et le résidu d'évaporation est chromatographié sur colonne de silice en éluant avec un mélange dichlorométhane-méthanol (95-5 en volumes), puis avec du méthanol seul. L'éluat au méthanol (600 mg) est purifié sur colonne de silice en éluant avec un mélange dichlorométhane-méthanol (90-10 en volumes). On obtient 400 mg de 1-(3-diéthylaminopropionyl)-spiro[pipéridine-4,10'-5'H,10'H-imidazo[1,2-a]indéno[1,2-e]pyrazine]-4'-one sous forme de meringue beige [Spectre de masse: (D/Cl; gaz réactif: ammoniac): M/Z= 420 (MH⁺)].

Le chlorure de 3-diméthylaminopropionyle peut être obtenu comme décrit dans le brevet DE 2550566.

### EXEMPLE 15

A une solution agitée de 1 g de bromhydrate de spiro[pipéridine-4,10'-5'H,10'H-imidazo[1,2-a]indéno[1,2-e]pyrazine]-4'-one dans 25 ml de diméthylformamide on ajoute 0,38 ml de triéthylamine et on poursuit l'agitation pendant 30 minutes avant d'ajouter goutte à goutte 0,32 ml d'isocyanate de méthyle. L'agitation est poursuivie durant la nuit à une température voisine de 20°C. Le milieu réactionnel est filtré et le solide obtenu est lavé au diméthylformamide, puis à l'eau distillée et enfin à l'éther éthylique. Après séchage à 35°C sous vide (1mmHg; 0,13 kPa) on obtient 250 mg de 1-(méthylcarbamoyl)-spiro[pipéridine-4,10'-5'H,10'H-imidazo[1,2-a]indéno [1,2-e]pyrazine]-4'-one sous forme de solide blanc fondant au-dessus de 260°C (Analyse C₁₉H₁₉N₅O₂ % calculé C : 65,32, H : 5,48, N : 20,04, O : 9,16, % trouvé C : 65,3, H : 5,5, N : 19,8, O : 9,5).

### EXEMPLE 16

A un mélange agité de 1,1 g de bromhydrate de spiro[pipéridine-4,10'-5'H,10'H-imidazo[1,2-a]indéno[1,2-e]pyrazine]-4'-one dans 60 ml de diméthylformamide on ajoute 1 ml de triéthylamine et on continue d'agiter pendant 30 minutes. On ajoute alors 0,15 ml d'iodure de méthyle et l'on poursuit l'agitation durant la nuit à une température voisine de 20°C. Le mélange réactionnel est additionné de 30 ml d'eau et filtré. Le solide obtenu est lavé à l'acétone, puis avec un mélange d'acétone et d'eau distillée et ensuite séché à l'air pour donner un produit beige (470 mg). Ce produit brut est purifié sur une colonne de silice en éluant avec un mélange dichlorométhane-méthanol (80-20 en volumes). On obtient 310 mg qui sont repris dans l'acétate d'éthyle bouillant (50 ml). Après filtration à chaud le filtrat fournit par cristallisation 109 mg de 1-méthyl-spiro[pipéridine-4,10'-5'H,10'H-imidazo[1,2-a]indéno[1,2-e]pyrazine]-4'-one sous forme de solide blanc cassé fondant vers 232°C avec décomposition [Spectre de R.M.N.: (300 MHz; DMSO d6 + CD₃CO₂D; δ en ppm): 1,60 (1H, d, J = 14 Hz, CH éq.), 2,77 (1H, m, CH ax.), 3,01 (3H, s, NCH₃), 3,50 (1H, t, J = 14 Hz, NCH ax.), 3,60 (1H, m, NCH éq.), 7,40 (1H, t, J = 7 Hz, CH arom.), 7,53 (1H, t, J = 7 Hz, CH arom.), 7,73 (1H, s, CH arom.), 8,00 (1H, d, J = 7 Hz, CH arom.), 8,22 (1H, d, J = 7 Hz, CH arom.), 8,40 (1H, s CH arom.)].

### EXEMPLE 17

On opère comme à l'exemple 16 mais à partir de 746 mg de bromhydrate de spiro[pipéridine-4,10'-5'H,10'H-imidazo[1,2-a]indéno[1,2-e]pyrazine]-4'-one, 40 ml de diméthylformamide, 0,6 ml de triéthylamine et 0,23 ml de chlorure de benzyle. Le mélange réactionnel est concentré au rotavapor et le résidu d'évaporation est trituré avec un mélange d'eau et de dichlorométhane, puis filtré. Le solide obtenu est lavé à l'eau distillée, à l'éther éthylique, puis de nouveau à l'eau distillée et à l'éther éthylique. Après séchage à 35°C sous vide (1 mmHg; 0,13 kPa) on obtient 440 mg de 1-benzyl-spiro[pipéridine-4,10'-5'H,10'H-imidazo[1,2-a]indéno[1,2-e]pyrazine]-4'-one sous forme de sel gris clair fondant au-dessus de 260°C (Analyse C₂₄H₂₂N₄O, 0,5 HBr, HCl % calculé C : 62,75, H : 5,16, Br: 8,70, CI: 7,72, N : 12,20, O : 3,48, % trouvé C : 62,8, H : 5,3, N : 12,3, O : 3,7).

### EXEMPLE 18

On opère comme à l'exemple 16 mais à partir de 746 mg de bromhydrate de spiro[pipéridine-4,10'-5'H,10'H-imidazo[1,2-a]indéno[1,2-e]pyrazine]-4'-one, 40 ml de diméthylformamide, 0,6 ml de triéthylamine et 0,26 ml de 12-chloroéthyl)benzène et en complétant le temps d'agitation du mélange réactionnel par 6 heures de reflux. Le milieu réactionnel est filtré et la phase organique est évaporée au rotavapor. Le résidu d'évaporation est trituré avec un mélange d'eau et de dichlorométhane et filtré. Le solide obtenu est lavé à l'eau distillée, puis à l'éther éthylique (3x20 ml) et purifié par chromatographie sur colonne de silice en éluant avec un mélange dichlorométhane-méthanol (95-5 en volumes). Le solide obtenu (330 mg) est trituré dans l'éther isopropylique, puis dans l'éther éthylique. Après séchage à 40°C sous vide (1 mmHg; 0,13 kPa) on obtient 140 mg de 1-phénéthyl-spiro[pipéridine-4,10'-5'H,10'H-imidazo[1,2-a]indéno[1,2-e]pyrazine]-4'-one sous forme de sel beige fondant au-dessus de 260°C (Analyse C₂₅H₂₄N₄O, HBr, HCl % calculé C : 58,43, H : 5,10, Br: 15,55, Cl: 6,90, N : 10,90, O : 3,11, % trouvé C : 58,1, H : 4,8, N : 11,0, O : 2,9).

### EXEMPLE 19

On procède comme à l'exemple 16 mais à partir de 1 g de bromhydrate de spiro[pipéridine-4, 10'-5'H,10'H-im idazo[1,2-a]indéno[1,2-e]pyrazine]-4'-one, 30 ml de diméthylformamide, 0,75 ml de triéthylamine et 0,4 ml de 1-chloro-3-phénylpropane et en complétant le temps d'agitation de milieu réactionnel par 6 heures de reflux. Le mélange réactionnel est concentré au rotavapor et le résidu d'évaporation est trituré avec de l'éther isopropylique et filtré. Le solide brun obtenu est purifié par chromatographie sur colonne de silice en éluant avec un mélange dichlorométhane-méthanol (95-5 en volumes). Après trituration dans l'éther éthylique on obtient un solide marron (200 mg) que l'on chromatographie sur un colonne de silice en éluant avec un mélange dichlorométhane-méthanol (90-10 en volumes). Après trituration dans l'éther éthylique, filtration et séchage, on obtient 80 mg de 1-(3-phénylpropyl)-spiro[pipéridine-4, 10'-5'H,10'H-imidazo[1,2-a]indéno[1,2-e]pyrazine]-4'-one sous forme de solide beige fondant vers 215°C avec décomposition [Spectre de R.M.N.: (400 MHz; DMSO d6; δ en ppm): 1,35 (2H, d, J = 13Hz, 2HCH pipèridine), 1,90 (2H, m, CH₂), 2,58 (4H, m, 2HCH et NCH₂), 2,75 (4H, m, NCH₂ et 2 HCHN), 3,03 (2H, d, J = 10Hz, 2 HCHN), 7,22 (2H, t, J = 7Hz, CH phényl), 7,33 (4H, m, 1 CH arom. et 3 CH phényl), 7,45 (1H, t, J = 7Hz, CH arom.), 7,65 (1H, s, CH arom.), 7,95 (1H, d, J = 7Hz, CH arom.), 8,00 (1H, s, CH arom.), 8,03 (1H, d, J = 7Hz, CH arom.), 12,4 (1H, s, NH).

### EXEMPLE 20

A une suspension agitée de 2,56 g de (10'RS)-spiro[pipéridine-2,10'-5'H,10'H-imidazo[1,2-a]indéno[1,2-e]pyrazine]-4'-one dans 40 ml d'acide acétique on ajoute 2,57 g d'anhydride succinique et on poursuit l'agitation pendant 48 heures à une température voisine de 20°C , puis à une température de 80°C pendant 2 heures. Le mélange réactionnel est concentré au rotavapor et le résidu d'évaporation est additionné de 100 ml d'éthanol absolu. La suspension obtenue est filtrée, le solide est lavé à l'éthanol (2x3 ml) et séché à 60°C sous pression réduite. Une partie (0,5 g) du solide obtenu est purifié par chromatographie sur colonne de silice en éluant avec un mélange dichlorométhane-méthanol-ammoniaque à 25% (12-6-1 en volumes). Après trituration dans l'éther isopropylique (5 ml), filtration et séchage à 60°C sous vide (1 mmHg; 0,13 kPa), on obtient 0,13 g d'acide (10'RS)-4-oxo-4-{4'-oxo-4',5'-dihydro-spiro[pipéridine-2, 10'-10'H-imidazo[1,2-a]indéno[1,2-e]pyrazine-1-yl]}butyrique sous forme de solide crème fondant vers 220°C avec décomposition (Analyse C₂₁H₂₀N₄O₄ % calculé C : 64,28, H : 5,14, N : 14,28, O : 16,31, % trouvé C : 63,9, H : 4,7).

### EXEMPLE 21

A une solution agitée de 0,89 g de (10'RS)-spiro[pipéridine-2,10'-5'H,10'H-imidazo[1,2-a]indéno[1,2-e]pyrazine]-4'-one dans 1,15 ml d'acide formique on ajoute 2,3 ml de formaldéhyde à 37% et on poursuit l'agitation pendant 2 heures à une température voisine de 20°C, puis pendant 45 minutes à une température comprise entre 30 et 40°C. Le mélange réactionnel est concentré au rotavapor et le résidu est additionné de 10 ml d'éthanol absolu et filtré. Le filtrat est évaporé au rotavapor et le résidu d'évaporation est trituré avec 10 ml d'eau distillée chaude et filtré. Le solide obtenu est lavé à l'eau distillée (3x5 ml), puis à l'acétone (2x5 ml). Après séchage à 60°C sous vide (1 mmHg; 0,13 kPa) on obtient 0,25 g de (10'RS)-1-méthyl-spiro[pipéridine-2,10'-5'H,10'H-imidazo[1,2-a]indéno[1,2-e]pyrazine]-4'-one sous forme de solide crème fondant vers 240°C avec décomposition (Analyse C₁₈H₁₈N₄O % calculé C : 70,57, H : 5,92, N : 18,29, O : 5,22, % trouvé C : 70,7, H : 5,5, N:18,2).

### EXEMPLE 22

On opère comme à l'exemple 3 mais à partir de 3,9 g de 10-acétylamino-5H,10H-imidazo[1,2-a]indéno[1,2-e]pyrazine]-4-one, 43 ml de diméthylsulfoxyde, 1,7 g d'hydrure de sodium à 80% et 1,5 ml de 1-bromo-3-chloropropane. Après hydrolyse, le milieu réactionnel est soumis à des extractions à l'acétate d'éthyle (4x300 ml) et la phase aqueuse est évaporée au rotavapor. Le résidu gommeux est trituré avec 30 ml d'eau distillée, filtré, lavé à l'eau distillée (5 ml) et séché à l'air pour donner un solide gris verdâtre (2,3 g). Une partie (1,3 g) de ce produit est additionnée de 160 ml d'eau distillée et le mélange est chauffé à reflux pendant 5 minutes, puis filtré à chaud. Le filtrat est laissé dans un bain d'eau et de glace pendant une heure et le précipité qui apparaît est filtré, lavé à l'eau distillée (2x5 ml) et séché à 60°C sous vide (1 mmHg; 0,13 kPa). On obtient 0,52 g de (10'RS)-1-acétyl-spiro[pyrrolidine-2,10'-5'H,10'H-imidazo[1,2-a]indéno[1,2-e]pyrazine]-4'-one sous forme de solide gris clair fondant à 270°C avec décomposition (Analyse C₁₈H₁₆N₄O₂ % calculé C : 67,49, H : 5,03, N : 17,49, O: 9,99, % trouvé C : 67,3, H : 5,3, N : 17,6).

La 10-acétylamino-5H,10H-imidazo[1,2-a]indéno[1,2-e]pyrazine]-4-one peut être obtenue comme décrit dans le brevet FR 2707643.

### EXEMPLE 23

A une suspension agitée de 4,65 g de (10'RS)-1-phtalimidoacétyl-spiro[pyrrolidine-2,10'-5'H,10'H-imidazo[1,2-a]indéno[1,2-e]pyrazine]-4'-one dans 10 ml de méthanol on ajoute 2 ml d'hydrate d'hydrazine et on chauffe le mélange à reflux pendant 16 heures. Après retour à une température voisine de 20°C, la suspension obtenue est filtrée et le solide est lavé au méthanol (3x30 ml). Le filtrat méthanolique est évaporé au rotavapor et le résidu d'évaporation est purifié par chromatographie sur colonne de silice en éluant avec un mélange dichlorométhane-méthanol-ammoniaque à 25% (70-30-3 en volumes). Après lavages à l'acétate d'éthyle (4x20 ml), puis à l'éther isopropylique (4x30 ml) on obtient 1,7 g de (10'RS)-1-glycyl-spiro[pyrrolidine-2,10'-5'H,10'H-imidazo[1 ,2-a]indéno[1,2-e]pyrazine]-4'-one sous forme de solide jaune crème se décomposant vers 290°C (Analyse C₁₈H₁₇N₅O₂ % calculé C : 64,47, H : 5,11, N : 20,88, O : 9,54, % trouvé C : 64,4, H : 4,7, N : 21,0).

La (10'RS)-1-phtalimidoacétyl-spiro[pyrrolidine-2,10'-5'H,10'H-imidazo[1,2-a]indéno[1,2-e]pyrazine]-4'-one peut être obtenue de la manière suivante : A une solution agitée de 1,1 g de spiro[pyrrolidine-2,10'-5'H,10'H-imidazo[1,2-a]indéno[1,2-e]pyrazine]-4'-one dans 45 ml de diméthylformamide on ajoute 0,56 ml de triéthylamine, puis en 5 minutes une solution de 0,89 g de chlorure de N-phtaloylglycyle dans 10 ml de diméthylformamide. On poursuit l'agitation pendant 16 heures à une température voisine de 20°C. Le milieu réactionnel est évaporé au rotavapor et le résidu est trituré à l'eau distillée (3x40 ml), filtré et séché à l'air. Une partie (0,31 g) du produit brut obtenu (1,54 g) est trituré dans l'acétate d'éthyle (4x50 ml), puis dans l'éther isopropylique (4x40 ml), et séché à 60°C sous vide (1 mmHg; 0,13 kPa). On obtient 0,28 g de (10'RS)-1-phtalimidoacétyl-spiro[pyrrolidine-2,10'-5'H,10'H-imidazo[1,2-a]indéno[1,2-e]pyrazine]-4'-one sous forme de solide marron clair se décomposant vers 350°C (Analyse C₂₆H₁₉N₅O₄ % calculé C : 67,09, H : 4,11, N : 15,05, O : 13,75, % trouvé C : 67,1, H : 4,1, N : 15,0).

### EXEMPLE 24

A une solution agitée de 1 g de (10'RS)-spiro[pyrrolidine-2,10'-5'H,10'H-imidazo[1,2-a]indéno[1,2-e]pyrazine]-4'-one dans 50 ml de diméthylformamide, on ajoute 0,23 ml d'isocyanate de méthyle et on continue l'agitation pendant 2 heures à une température voisine de 20°C. Après évaporation du mélange réactionnel, le résidu est trituré dans 50 ml d'acétone, filtré, lavé à l'acétone (3x5 ml) et séché à 60°C sous vide (1 mmHg; 0,13 kPa). On obtient 1,15 g de (10'RS)-1-(méthylcarbamoyl)-spiro[pyrrolidine-2,10'-5'H,10'H-imidazo[1,2-a]indéno[1,2-e]pyrazine]-4'-one sous forme de solide de couleur crème se décomposant vers 270°C (Analyse C₁₈H₁₇N₅O₂ % calculé C : 64,47, H : 5,11, N : 20,88, O : 9,54, % trouvé C : 64,4, H : 4,9, N : 20,8).

### EXEMPLE 25

On opère comme à l'exemple 24 mais à partir de 1 g de (10'RS)-spiro[pyrrolidine-2,10'-5'H,10'H-imidazo[1,2-a]indéno[1,2-e]pyrazine]-4'-one, 50 ml de diméthylformamide et 0,44 ml d'isocyanate de phényle. On obtient 1,1 g de (10'RS)-1-(phénylcarbamoyl)-spiro[pyrrolidine-2,10'-5'H,10'H-imidazo[1,2-a]indéno[1,2-e]pyrazine]-4'-one sous forme de solide blanc se décomposant vers 280°C (Analyse C₂₃H₁₉N₅O₂ % calculé C : 69,51, H : 4,82, N : 17,62, O : 8,05, % trouvé C : 69,5, H : 4,9, N : 17,3, O : 7,7).

### EXEMPLE 26

On opère comme à l'exemple 24 mais à partir de 1 g de (10'RS)-spiro[pyrrolidine-2,10'-5'H, 10'H-imidazo[1,2-a]indéno[1,2-e]pyrazine]-4'-one, 50 ml de diméthylformamide et 0,49 ml d'isocyanate de benzyle. On obtient 1,3 g de (10'RS)-1-(benzylcarbamoyl)-spiro[pyrrolidine-2,10'-5'H,10'H-imidazo[1,2-a]indéno[1,2-e]pyrazine]-4'-one sous forme de solide blanc fondant à 240°C, puis se décomposant (Analyse C₂₄H₂₁N₅O₂ % calculé C : 70,06, H : 5,14, N : 17,02, O : 7,78, % trouvé C : 69,8, H : 5,4, N : 17,0).

### EXEMPLE 27

On procède comme à l'exemple 6 mais à partir de 0,85 g de (10'RS)-8'-fluoro-spiro[pyrrolidine-2, 10'-5'H,10'H-imidazo[1,2-a]indéno[1,2-e]pyrazine]-4'-one, 2,13 ml de formaldéhyde à 37% et 1,1 ml d'acide formique. Après évaporation du mélange réactionnel, le résidu est chromatographié sur colonne de silice en éluant avec un mélange dichlorométhane-méthanol (90-10 en volumes). Le solide jaune obtenu (0,61 g) est additionné de 150 ml de méthanol; le mélange est porté à reflux pendant 10 minutes et filtré à chaud. Le filtrat est évaporé au rotavapor et le résidu d'évaporation est trituré avec 30 ml d'éther éthylique, filtré et séché à 60°C sous vide (1 mmHg; 0,13 kPa). On obtient 0,41 g de (10'RS)-1-méthyl-8'-fluoro-spiro[pyrrolidine-2,10'-5'H,10'H- imidazo[1,2-a]indéno[1,2-e]pyrazine]-4'-one sous forme de solide blanc fondant vers 310°C avec décomposition (Analyse C₁₇H₁₅FN₄O % calculé C : 65,80, H : 4,87, F: 6,12, N : 18,05, O: 5,16, % trouvé C : 65,9, H : 4,6, F: 6,0, N : 17,8).

La (10'RS)-8'-fluoro-spiro[pyrrolidine-2,10'-5'H,10'H-imidazo[1,2-a]indéno [1,2-e]pyrazine]-4'-one peut être préparée de la manière suivante : on opère comme à l'exemple 3 mais à partir de 2,37 g de 10-tert-butoxycarbonylam ino-8-fluoro-5H,10H-imidazo[1,2-a]indéno[1,2-e]pyrazine]-4-one, 22,5 ml de diméthylsulfoxyde, 0,81 g d'hydrure de sodium à 80% et 0,72 ml de 1-bromo-3-chloropropane. On obtient intermédiairement 2,2 g de (10'RS)-1-tert-butoxycarbonylamino-8'-fluoro-spiro[pyrrolidine-2,10'-5'H,10'H-imidazo[1,2-a]indéno[1,2-e]pyrazine]-4'-one sous forme de solide blanc fondant au-dessus de 260°C (Rf=0,57, chromatographie sur couche mince de gel de silice, éluant : chloroforme-méthanol-ammoniaque à 28% (12-3-0,5 en volumes)). La déprotection du NH a été effectuée sur 0,88 g de (10'RS)-1-tert-butoxycarbonylamino-8'-fluoro-spiro[pyrrolidine-2,10'-5'H,10'H-imidazo [1,2-a]indéno[1,2-e]pyrazine]-4'-one à l'aide de 9 ml d'acide trifluoroacétique. On obtient 0,45 g de (10'RS)-8'-fluoro-spiro[pyrrolidine-2,10'-5'H,10'H-imidazo[1,2-a]indéno[1,2-e]pyrazine]-4'-one sous forme de solide beige se décomposant vers 260°C (Rf=0,50, chromatographie sur couche mince de gel de silice, éluant : chloroforme-méthanol-ammoniaque à 28% (12-3-0,5 en volumes)).

La 10-tert-butoxycarbonylamino-8-fluoro-5H,10H-imidazo[1,2-a]indéno[1,2-e]pyrazine]-4-one peut être préparée de la façon suivante : on procède comme à l'exemple 3 mais à partir de 0,44 g de 10-amino-8-fluoro-5H,10H-imidazo[1,2-a]indéno[1,2-e]pyrazine]-4-one, 5,5 ml de diméthylformamide, 0,26 ml de triéthylamine et 0,5 g de dicarbonate de di-tert-butyle. On obtient 0,16 g de 10-tert-butoxycarbonylamino-8-fluoro-5H,10H-imidazo[1,2-a]indéno [1,2-e]pyrazine]-4-one sous forme de solide blanc écru fondant au-dessus de 260°C (Rf=0,53, chromatographie sur couche mince de gel de silice, éluant : chloroforme-méthanol-ammoniaque à 28% (12-3-0,5 en volumes)).

La 10-amino-8-fluoro-5H,10H-imidazo[1,2-a]indéno[1,2-e]pyrazine]-4-one peut être obtenue comme décrit dans le brevet FR 2707643.

### EXEMPLE 28

Une solution agitée de 778 mg de (10'RS)-spiro[pyrrolidine-2,10'-5'H,10'H-imidazo[1,2-a]indéno[1,2-e]pyrazine]-4'-one dans 5 ml d'acide acétique est chauffée à une température voisine de 45°C et additionnée en 1 heure de 510 mg de borohydrure de sodium. L'agitation est maintenue à cette température pendant 16 heures. Le milieu réactionnel est ensuite versé dans 50 ml d'eau distillée et laissé au repos pendant 1 heure à une température voisine de 20°C. Le précipité formé est filtré, lavé à l'eau distillée (3x30 ml) et séché à l'air. Le produit brut est purifié par chromatographie sur colonne de silice en éluant avec de l'acétate d'éthyle. Après séchage à 60°C sous vide (1 mmHg; 0,13 kPa) on obtient 230 mg de (10'RS)-1-éthyl-spiro[pyrrolidine-2,10'-5'H,10'H-imidazo[1,2-a]indéno[1,2-e]pyrazine]-4'-one sous forme de solide blanc fondant à 228°C (Analyse C₁₈H₁₈N₄O % calculé C : 70,57, H : 5,92, N : 18,29, O : 5,22, % trouvé C : 70,6, H : 6,1, N : 18,3).

### EXEMPLE 29

On opère comme à l'exemple 28 mais à partir de 778 mg de (10'RS)-spiro[pyrrolidine-2,10'-5'H,10'H-imidazo[1,2-a]indéno[1,2-e]pyrazine]-4'-one, 6,5 ml d'acide propionique et 510 mg de borohydrure de sodium. Le produit brut (740 mg) est purifié par cristallisation dans 25 ml d'acétate d'éthyle. On obtient 430 mg de (10'RS)-1-propyl-spiro[pyrrolidine-2,10'-5'H,10'H-imidazo [1,2-a]indéno[1,2-e]pyrazine]-4'-one sous forme de solide jaune pâle fondant à 248°C (Analyse C₁₉H₂₀N₄O % calculé C : 71,23, H : 6,29, N:17,49, O : 4,99, % trouvé C : 71,5, H : 6,4, N : 17,4, O : 5,2).

### EXEMPLE 30

A une suspension agitée de 1 g de (10'RS)-spiro[pyrrolidine-2,10'-5'H,10'H-imidazo[1,2-a]indéno[1,2-e]pyrazine]-4'-one dans 50 ml d'éthanol absolu on ajoute 0,45 g de potasse en pastilles. La solution jaune orangé obtenue est additionnée de 1 ml de bromure de benzyle et agitée à une température voisine de 20°C pendant la nuit. Le mélange réactionnel est évaporé au rotavapor et le résidu d'évaporation est trituré dans l'acétate d'éthyle (3x40 ml) et filtré. Le solide est traité à l'acide acétique et concentré au rotavapor. La meringue obtenue est purifiée sur colonne de silice en éluant avec un mélange d'acétate d'éthyle et de méthanol (90-10 en volumes). On obtient 73 mg de (10'RS)-1-benzyl-spiro[pyrrolidine-2,10'-5'H,10'H-imidazo[1,2-a]indéno[1,2-e]pyrazine]-4'-one sous forme de solide beige [Spectre de R.M.N.: (300 MHz; DMSO d6; δ en ppm) : 2,05 et 2,65 (1 H chacun, m, CH₂), 2,35 (2H, m, CH₂), 3,12 et 3,40 (1H chacun, m, NCH₂), 3,15 et 3,23 (1H chacun, m, NCH₂ Ph), 6,95 (2H, t, J=7 Hz, CH phényl), 7,02 (3H, m, CH phényl), 7,40 (1H, t, J=7 Hz, CH arom.), 7,47 (1H, t, J=7 Hz, CH arom.), 7,60 (1H, d, J=7 Hz, CH arom.), 7,65 (1H, s, CH arom.), 7,89 (1H, d, J=7 Hz, CH arom.), 8,10 (1H, s, CH arom.), 12,3 (1H, s, NH).

Les médicaments selon l'invention sont constitués par un composé de formule (I) ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention des conditions qui requièrent l'administration d'un antagoniste du récepteur AMPA ou d'un antagoniste du récepteur NMDA. Ces composés sont notamment utiles pour traiter ou prévenir toutes les ischémies et en particulier l'ischémie cérébrale, les effets dus à une anoxie, l'évolution de maladies neurodégénératives, de la chorée d'HUNTINGTON, de la maladie d'ALZHEIMER, de la sclérose latérale amyotrophique, de l'atrophie olivo-pontocérébelleuse et de la maladie de PARKINSON, vis-à-vis des manifestations épileptogènes et/ou convulsives, pour le traitement des traumatismes cérébraux et spinaux, des traumatismes liés à le dégénérescence de l'oreille interne ou de la rétine, de l'anxiété, de la dépression, de la schizophrénie, du syndrome de TOURETTE, de l'encéphalopathie hépatique, en tant qu'analgésiques, antiinflammatoires, antianorexiques, antimigraineux, antiémétiques et pour traiter les empoisonnements par des neurotoxines ou d'autres substances agonistes du récepteur NMDA, ainsi que les troubles neurologiques associés aux maladies virales telles que le sida, la rage, la rougeole et le tétanos. Ces composés sont aussi utiles pour la prévention des symptômes d'abstinence aux drogues et à l'alcool et de l'inhibition de l'accoutumance et de la dépendance aux opiacés ainsi que pour le traitement des déficits liés à des anomalies mitochondriales tels que la myopathie mitochondriale, le syndrome de LEBER, l'encéphalopathie de WERNICKE, le syndrome de RETT, l'homocystéinémie, l'hyperprolinémie, l'hydroxybutirique-aminoacidurie, l'encéphalopathie saturnine (intoxication chronique au plomb) et la déficience en sulfite oxydase.

Les doses dépendent de l'effet recherché, de la durée du traitement et de la voie d'administration utilisée; elles sont généralement comprises entre 10 mg et 100 mg par jour par voie orale pour un adulte avec des doses unitaires allant de 5 mg à 50 mg de substance active.

D'une façon générale, le médecin déterminera la posologie appropriée en fonction de l'age, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

## Revendications

1. Composés de formule : 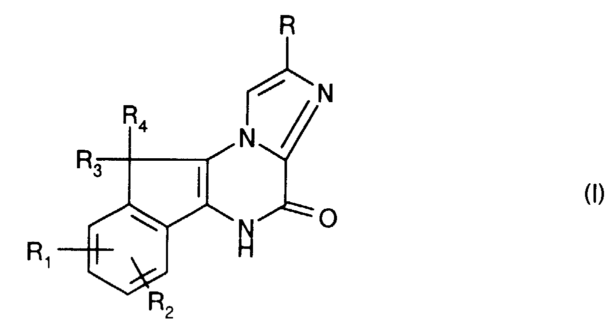 dans laquelle
- R représente un atome d'hydrogène ou un radical carboxy, alcoxycarbonyle ou carboxamido,
- R₁ et R₂, identiques ou différents, représentent des atomes d'hydrogène ou d'halogène ou des radicaux alkyle, alcoxy, amino, -N=CH-N(alk)alk', nitro, cyano, phényle, imidazolyle, SO₃H, hydroxy, polyfluoroalcoxy, carboxy, alcoxycarbonyle, -NH-CO-NR₅R₆, -N(alk)-CO-NR₅R₆, -N(alk-Ar)-CO-NR₅R₆, -NH-CS-NR₅R₆, -Nlalk)-CS-NR₅R₆, -NH-CO-R₅, -NH-CS-R₇, -NH-C(=NR₉)-NR₈R₆, -N(alk)-C(=NR₉)-NR₈R₆, -CO-NR₈R₆, -NH-SO₂-NR₈R₆, -N(alk)-SO₂-NR₈R₆, -NH-SO₂-CF₃, -NH-SO₂-alk, -NH-SO₂-Ar, -NR₈R₁₀, -S(O)ₘ-alk-Ar, -SO₂-NR₈R₆ ou 2-oxo-1-imidazolidinyle dont la position -3 est éventuellement substituée par un radical alkyle ou 2-oxo-1-perhydropyrimidinyle dont la position -3 est éventuellement substituée par un radical alkyle,
- R₃ et R₄ forment ensemble avec l'atome de carbone auquel ils sont rattachés un cycle 2- ou 3-pyrrolidine, un cycle 2- ou 4-pipéridine, un cycle 2-azacycloheptane, ces cycles étant éventuellement substitués sur l'azote par un radical alkyle, -CHO, -COOR₁₁, -CO-alk-COOR₆, -CO-alk-NR₆R₁₂, -CO-alk-CONR₆R₈, -CO-COOR₆, -CO-CH₂-O-CH₂-COOR₆, -CO-CH₂-S-CH₂-COOR₆, -CO-CH=CH-COOR₆, -CO-alk, -CO-Ar", -CO-alk-Ar", -CO-NH-Ar", -CO-NH-alk-Ar", -CO-Het, -CO-alk-Het, -CO-NH-Het, -CO-NH-alk-Het, -CO-NH₂, -CO-N H-alk, -CO-N(alk)alk', -CS-NH₂, -CS-NH-alk, -CS-NH-Ar", -CS-NH-Het, -alk-Het, -alk-NR₆R₈, -alk-COOR₆, -alk-CO-NR₆R₈, -alk-Ar", -SO₂-alk, -SO₂-Ar ou -CO-cycloalkyle dont le cycloalkyle est éventuellement substitué en -2 par un radical carboxy,
- R₅ représente un atome d'hydrogène ou un radical alkyle (1-9C en chaîne droite ou ramifiée), -alk-COOR₈, -alk-Het, -alk-NR₆R₈, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, carboxy, alcoxycarbonyle, cyano et -alk-COOR₈, phényle éventuellement substitué par un ou plusieurs substituants choisis parmi les atomes d'halogène et les radicaux alkyle, alcoxy, nitro, amino, hydroxy, -alk-NH₂, carboxy, alcoxycarbonyle, cyano et -alk-COOR₈ ou -Het,
- R₆ représente un atome d'hydrogène ou un radical alkyle,
- R₇ représente un radical alkyle ou phényle,
- R₈ représente un atome d'hydrogène ou un radical alkyle,
- R₉ représente un atome d'hydrogène ou un radical alkyle,
- R₁₀ représente un radical alkyle, Het ou alcoxycarbonyle,
- R₁₁ représente un radical alkyle ou phénylalkyle,
- R₁₂ représente un atome d'hydrogène ou un radical alkyle,
- alk représente un radical alkyle ou alkylène,
- alk' représente un radical alkyle,
- m est égal à 0, 1 ou 2,
- Ar représente un radical phényle,
- Ar" est un radical phényle ou phényle substitué par un ou plusieurs substituants choisis parmi les atomes d'halogène et les radicaux alkyle, alcoxy, ni-tro, amino, hydroxy, cyano, -alk-NH₂, COOR₆ et -alk-COOR₆,
- Het représente un hétérocycle mono ou polycyclique saturé ou insaturé contenant 1 à 9 atomes de carbone et un ou plusieurs hétéroatomes (O, S, N) éventuellement substitué par un ou plusieurs radicaux alkyle, phényle, carboxy ou phénylalkyle,
étant entendu que sauf mention contraire les radicaux et portions alkyle, alkylène et alcoxy contiennent 1 à 6 atomes de carbone et sont en chaîne droite ou ramifiée et les radicaux cycloalkyle contiennent 3 à 6 atomes de carbone,
les énantiomères et diastéréoisomères des composés de formule (I) pour lesquels R₃ et R₄ forment ensemble avec l'atome de carbone auquel ils sont rattachés un cycle 2- ou 3-pyrrolidine, 2-pipéridine, ou 2-azacycloheptane, les isomères cis et trans des composés de formule (I) comportant un radical -CO-CH=CH-COOR₆
et les sels de ces composés.

2. Composés de formule (I) selon la revendication 1 pour lesquels Het est choisi parmi les cycles pyrrolyle éventuellement substitué par un ou plusieurs radicaux alkyle, phényle, carboxy ou phénylalkyle, pyridyle éventuellement substitué par un ou plusieurs radicaux alkyle, phényle, carboxy ou phénylalkyle, pyrimidinyle éventuellement substitué par un ou plusieurs radicaux alkyle, phényle, carboxy ou phénylalkyle, imidazolyle éventuellement substitué par un ou plusieurs radicaux alkyle, phényle, carboxy ou phénylalkyle, thiazolyle éventuellement substitué par un ou plusieurs radicaux alkyle, phényle, carboxy ou phénylalkyle, oxazolinyle éventuellement substitué par un ou plusieurs radicaux alkyle, phényle, carboxy ou phénylalkyle, thiazolinyle éventuellement substitué par un ou plusieurs radicaux alkyle, phényle, carboxy ou phénylalkyle, pyrazinyle éventuellement substitué par un ou plusieurs radicaux alkyle, phényle, carboxy ou phénylalkyle, tétrazolyle éventuellement substitué par un ou plusieurs radicaux alkyle, phényle, carboxy ou phénylalkyle ou triazolyle éventuellement substitué par un ou plusieurs radicaux alkyle, phényle, carboxy ou phénylalkyle et leurs sels.

3. Composés de formule (I) selon la revendication (I) pour lesquels R représente un atome d'hydrogène ou un radical carboxy, alcoxycarbonyle ou carboxamido, R₁ et R₂ sont des atomes d'hydrogène, R₃ et R₄ forment ensemble avec l'atome de carbone auquel ils sont rattachés un cycle 2- ou 3-pyrrolidine, 2- ou 4-pipéridine, ces cycles étant éventuellement substitués sur l'azote par un radical alkyle ou -CO-alk-COOR₆ et leurs sels..

4. Composés suivants :
- (10'RS)-spiro[pyrrolidine-3,10'-5'H,10'H-imidazo[1,2-a]indéno[1,2-e]pyrazine]-4'-one,
- (10'RS)-spiro[pipéridine-2,10'-5'H,10'H-imidazo[1,2-a]indéno[1,2-e]pyrazine]-4'-one,
- (10'RS)-spiro[pyrrolidine-2,10'-5'H,10'H-imidazo[1,2-a]indéno[1,2-e]pyrazine]-4'-one,
- (10'RS)-1-méthyl-spiro[pyrrolidine-2,10'-5'H,10'H-imidazo[1,2-a]indéno[1,2-e]pyrazine]-4'-one,
- (+)-1-méthyl-spiro[pyrrolidine-2,10'-5'H,10'H-imidazo[1,2-a]indéno[1,2-e]pyrazine]-4'-one,
- (-)-1-méthyl-spiro[pyrrolidine-2,10'-5'H,10'H-imidazo[1,2-a]indéno[1,2-e]pyrazine]-4'-one,
- acide (10'RS)-4-oxo-4-(4'-oxo-4',5'-dihydro-spiro[pyrrolidine-2,10'-10'H-imidazo[1,2-a]indéno[1,2-e]pyrazine-1-yl]}-butyrique,
et leurs sels.

5. Procédé de préparation des composés de formule (I) selon la revendication 1 pour lesquels R₃ et R₄ forment avec l'atome de carbone auquel ils sont rattachés un cycle 2-pyrrolidine, 2-pipéridine ou 2-azacycloheptane caractérisé en ce que l'on fait réagir un dérivé de formule : 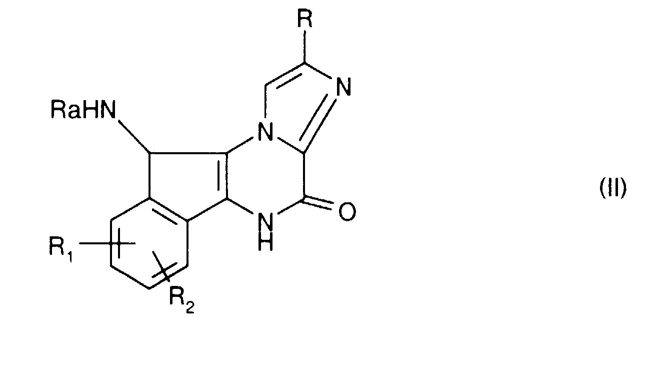 dans laquelle Ra représente un groupe protecteur de la fonction amine, R, R₁ et R₂ ont les mêmes significations que dans la revendication 1, sur un dérivé de formule :
Hal-(CH₂)ₚ-Hal' (III)
dans laquelle Hal et Hal', identiques ou différents, sont des atomes d'halogène et p est égal à 3, 4 ou 5 puis on déprotège le NH, isole le produit et le transforme éventuellement en sel.

6. Procédé de préparation des composés de formule (I) selon la revendication 1 pour lesquels R₃ et R₄ forment avec l'atome de carbone auquel ils sont rattachés un cycle 3-pyrrolidine caractérisé en ce que l'on fait réagir la N-n-butoxyméthyl-N-triméthylsilylméthyl-benzylamine sur un dérivé de formule : 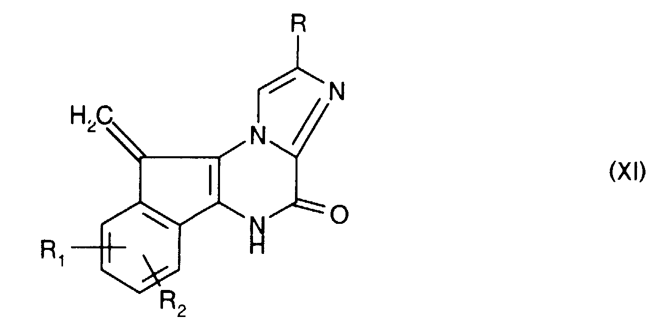 dans laquelle R, R₁ et R₂ ont les mêmes significations que dans la revendication 1 puis on débenzyle le NH, isole le produit et le transforme éventuellement en sel.

7. Procédé de préparation des composés de formule (I) selon la revendication 1 pour lesquels R₃ et R₄ forment avec l'atome d'azote auquel ils sont attachés un cycle 4-pipéridine caractérisé en ce que l'on fait réagir le N,N-bis(2-chloroéthyl)p-toluènesulfonamide sur un dérivé de formule : 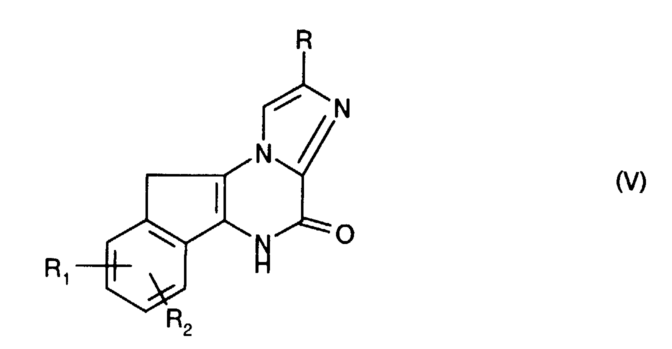 dans laquelle R, R₁ et R₂ ont les mêmes significations que dans la revendication 1 puis on hydrolyse la fonction sulfonamide, isole le produit et le transforme éventuellement en sel.

8. Procédé de préparation des composés de formule (I) selon la revendication 1 pour lesquels R₃ et R₄ forment avec l'atome de carbone auquel ils sont rattachés un cycle 2- ou 3-pyrrolidine, 2- ou 4-pipéridine ou 2-azacycloheptane dont l'atome d'azote est substitué par un radical alkyle 1C caractérisé en ce que l'on fait réagir le formaldéhyde et l'acide formique sur un composé de formule (I) correspondant pour lequel R₃ et R₄ forment avec l'atome de carbone auquel ils sont rattachés un cycle 2- ou 3-pyrrolidine, 2- ou 4-pipéridine ou 2-azacycloheptane, isole le produit et le transforme éventuellement en sel.

9. Procédé de préparation des composés de formule (I) selon la revendication 1 pour lesquels R₃ et R₄ forment avec l'atome de carbone auquel ils sont rattachés un cycle 2- ou 3-pyrrolidine, 2- ou 4-pipéridine ou 2-azacycloheptane dont l'atome d'azote est substitué par un radical alkyle (2-6C) caractérisé en ce que l'on fait réagir un acide alk-COOH dans lequel alk représente un radical alkyle 1-5C sur un composé de formule (I) correspondant pour lequel R₃ et R₄ forment avec l'atome de carbone auquel ils sont rattachés un cycle 2- ou 3-pyrrolidine, 2- ou 4-pipéridine ou 2-azacycloheptane, en présence de borohydrure de sodium, isole le produit et le transforme éventuellement en sel.

10. Procédé de préparation des composés de formule (I) selon la revendication 1 pour lesquels R₃ et R₄ forment avec l'atome de carbone auquel ils sont rattachés un cycle 2- ou 3-pyrrolidine, 2- ou 4-pipéridine ou 2-azacycloheptane dont l'atome d'azote est substitué par un radical -CO-alk-NR₆R₁₂ dans lequel R₆ et R₁₂ représentent chacun un atome d'hydrogène caractérisé en ce que l'on fait réagir un composé de formule (I) correspondant pour lequel R₃ et R₄ forment avec l'atome de carbone auquel ils sont rattachés un cycle 2- ou 3-pyrrolidine, 2- ou 4-pipéridine ou 2-azacycloheptane sur un dérivé de formule : 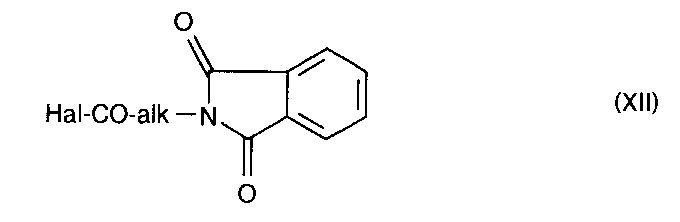 dans laquelle Hal représente un atome d'halogène et alk représente un radical alkyle puis déprotège le NH₂, isole le produit et le transforme éventuellement en sel.

11. Procédé de préparation des composés de formule (I) selon la revendication 1 pour lesquels R₃ et R₄ forment avec l'atome de carbone auquel ils sont rattachés un cycle 2- ou 3-pyrrolidine, 2- ou 4-pipéridine ou 2-azacycloheptane dont l'atome d'azote est substitué par un radical alkyle (2-6C), -COOR₁₁, -alk-Het, -alk-NR₆R₈, -alk-COOR₆, -alk-Ar", -CO-alk, -CO-alk-COOR₆, -CO-alk-NR₆R₁₂, -CO-COOR₆, -CO-cycloalkyle dont le cycloalkyle est éventuellement substitué en -2 par un radical carboxy, -CO-Ar", -CO-alk-Ar", -CO-Het, -CO-alk-Het, -CO-N(alk)alk', -SO₂-alk ou -SO₂-Ar caractérisé en ce que l'on fait réagir un composé de formule (I) correspondant pour lequel R₃ et R₄ forment avec l'atome de carbone auquel ils sont rattachés un cycle 2- ou 3-pyrrolidine, 2- ou 4-pipéridine ou 2-azacycloheptane sur un halogénure Hal-Rc dans lequel Hal représente un atome d'halogène et Rc représente un radical alkyle (2-6C), -COOR₁₁, -alk-Het, -alk-NR₆R₈, -alk-COOR₆, -alk-Ar", -CO-alk, -CO-alk-COOR₆, -CO-alk-NR₆R₁₂, -CO-COOR₆, -CO-cycloalkyle(3-6C) dont le cycloalkyle est éventuellement substitué en -2 par un radical carboxy, -CO-Ar", -CO-alk-Ar", -CO-Het, -CO-alk-Het, -CO-N(alk)alk', -SO₂-alk ou -SO₂-Ar, alk, alk', Het, R₆, R₈, R₁₁, R₁₂, Ar et Ar" ayant les mêmes significations que dans la revendication 1, isole le produit et le transforme éventuellement en sel.

12. Procédé de préparation des composés de formule (I) selon la revendication 1 pour lesquels R₃ et R₄ forment avec l'atome de carbone auquel ils sont rattachés un cycle 2- ou 3-pyrrolidine, 2- ou 4-pipéridine ou 2-azacycloheptane dont l'atome d'azote est substitué par un radical -CO-alk-CONR₆R₈ ou -alk-CO-NR₆R₈ caractérisé en ce que l'on fait réagir un composé de formule (I) correspondant pour lequel R₃ et R₄ forment avec l'atome de carbone auquel ils sont rattachés un cycle 2- ou 3-pyrrolidine, 2-ou 4-pipéridine ou 2-azacycloheptane dont l'atome d'azote est substitué par un radical -CO-alk-COOR₆ ou -alk-COOR₆ sur une amine HNR₆R₈ dans laquelle R₆ et R₈ ont les mêmes significations que dans la revendication 1, isole le produit et le transforme éventuellement en sel.

13. Procédé de préparation des composés de formule (I) selon la revendication 1 pour lesquels R₃ et R₄ forment avec l'atome de carbone auquel ils sont rattachés un cycle 2- ou 3-pyrrolidine, 2- ou 4-pipéridine ou 2-azacycloheptane dont l'atome d'azote est substitué par un radical -CHO caractérisé en ce que l'on fait réagir un composé de formule (I) correspondant pour lequel R₃ et R₄ forment avec l'atome de carbone auquel ils sont rattachés un cycle 2- ou 3-pyrrolidine, 2- ou 4-pipéridine ou 2-azacycloheptane sur CH₃COOCHO, isole le produit et le transforme éventuellement en sel.

14. Procédé de préparation des composés de formule (I) selon la revendication 1 pour lesquels R₃ et R₄ forment avec l'atome de carbone auquel ils sont rattachés un cycle 2- ou 3-pyrrolidine, 2- ou 4-pipéridine ou 2-azacycloheptane dont l'atome d'azote est substitué par un radical -CO-Het, -CO-alk-COOR₆, -CO-COOR₆, -CO-alk-NR₆R₁₂, -CO-Ar", -CO-alk-Ar", -CO-alk-Het, -CO-alk ou -CO-cycloalkyle dont le cycloalkyle est éventuellement substitué en -2 par un radical carboxy caractérisé en ce que l'on fait réagir un composé de formule (I) correspondant pour lequel R₃ et R₄ forment avec l'atome de carbone auquel ils sont rattachés un cycle 2- ou 3-pyrrolidine, 2- ou 4-pipéridine ou 2-azacycloheptane sur un dérivé HO-Rd dans lequel Rd représente un radical -CO-Het, -CO-alk-COOR₆, -CO-COOR₆, -CO-alk-NR₆R₁₂, -CO-Ar", -CO-alk-Ar", -CO-alk-Het, -CO-alk, -CO-cycloalkyle(3-6C) dont le cycloalkyle est éventuellement substitué en -2 par un radical carboxy, Het, alk, R₆, R₈, R₁₂, Het et Ar" ayant les mêmes significations que dans la revendication 1, isole le produit et le transforme éventuellement en sel.

15. Procédé de préparation des composés de formule (I) selon la revendication 1 pour lesquels R₃ et R₄ forment avec l'atome de carbone auquel ils sont rattachés un cycle 2- ou 3-pyrrolidine, 2- ou 4-pipéridine ou 2-azacycloheptane dont l'atome d'azote est substitué par un radical -CO-alk-COOR₆ dans lequel alk contient 1 à 3 atomes de carbone en chaîne droite, -CO-CH₂-C(CH₃)₂-CH₂-COOR₆, -CO-CH₂-CH₂-C(CH₃)₂-COOR₆, -CO-CH₂-C(CH₃)₂-COOR₆, -CO-CH₂-O-CH₂-COOR₆, -CO-CH₂-S-CH₂-COOR₆, -CO-CH=CH-COOR₆, -CO-cycloalkyle (6C) dont le cycloalkyle est substitué en -2 par un radical carboxy, -CO-Ar" dans lequel Ar" représente un radical phényle substitué en -2 par un radical carboxy, -CO-Het dans lequel Het représente un radical 2- ou 4-pyridyle substitué en position -3 par un radical carboxy ou 3-pyridyle substitué en position -4 par un radical carboxy et R₆ représente un atome d'hydrogène caractérisé en ce que l'on fait réagir un anhydride de formules : 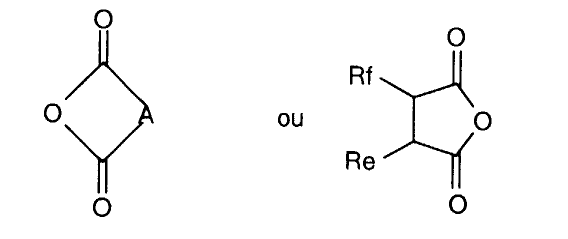 dans lesquelles A représente un radical alkyle (1-3C en chaîne droite), -CH₂-C(CH₃)₂-CH₂-, -CH₂-CH₂-C(CH₃)₂-, -CH₂-C(CH₃)₂-, -CH₂-O-CH₂-, -CH₂-S-CH₂, -CH=CH-, Re et Rf forment ensemble avec les 2 atomes de carbone auxquels ils sont rattachés un radical cycloalkyle(6C), phényle ou pyridyle sur un composé de formule (I) correspondant pour lesquels R₃ et R₄ forment avec l'atome de carbone auquel ils sont rattachés un cycle 2- ou 3-pyrrolidine, 2- ou 4-pipéridine ou 2-azacycloheptane, isole le produit et le transforme éventuellement en sel.

16. Procédé de préparation des composés de formule (I) selon la revendication 1 pour lesquels R₃ et R₄ forment avec l'atome de carbone auquel ils sont rattachés un cycle 2- ou 3-pyrrolidine, 2- ou 4-pipéridine ou 2-azacycloheptane dont l'atome d'azote est substitué par un radical -CO-NH-alk-Ar", -CO-NH-Het, -CO-NH-alk-Het, -CO-NH-Ar", -CO-NH-alk, -CO-NH₂, -CSNH₂, -CS-NH-alk, -CS-NH-Ar" ou -CS-NH-Het caractérisé en ce que l'on fait réagir un composé de formule (I) correspondant pour lequel R₃ et R₄ forment avec l'atome de carbone auquel ils sont rattachés un cycle 2- ou 3-pyrrolidine, 2- ou 4-pipéridine ou 2-azacycloheptane sur un dérivé de formule Rg=C=N-Rh dans lequel Rg représente un atome d'oxygène ou de soufre et Rh représente un radical triméthylsilyle, alkyle, Het, -alk-Ar", -alk-Het ou Ar", Het, alk et Ar" ayant les mêmes significations que dans la revendication 1, isole le produit et le transforme éventuellement en sel.

17. Procédé de préparation des composés de formule (I) selon la revendication 1 pour lesquels R représente un radical carboxy caractérisé en ce que l'on hydrolyse un composé de formule (I) correspondant pour lequel R représente un radical alcoxycarbonyle, isole le produit et le transforme éventuellement en sel.

18. Médicaments contenant en tant que principe actif au moins un composé de formule (I) selon la revendication 1.

19. Médicaments contenant en tant que principe actif au moins un composé de formule (I) selon l'une des revendications 2 à 4.

20. Médicaments selon l'une des revendications 18 et 19 utiles comme antagoniste du récepteur AMPA.

21. Médicaments selon l'une des revendications 18 et 19 utiles comme antagonistes du récepteur NMDA.

## Patentansprüche

1. Verbindungen der Formel 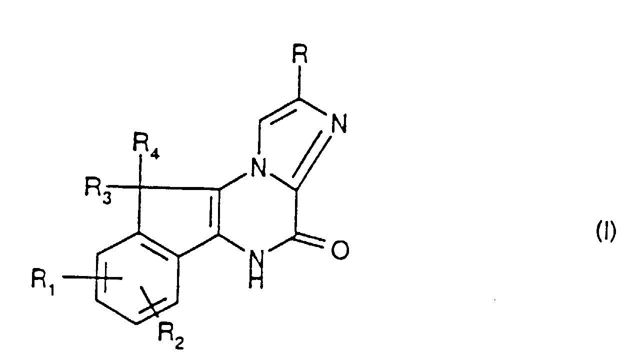 worin
- R ein Wasserstoffatom oder einen Carboxy-, Alkoxycarbonyl- oder Carboxamidorest bedeutet,
- R₁ und R₂, identisch oder verschieden, Wasserstoff- oder Halogenatome oder die Reste Alkyl, Alkoxy, Amino, -N=CH-N(alk)alk', Nitro, Cyano, Phenyl, Imidazolyl,SO₃H, Hydroxy, Polyfluoralkoxy, Carboxy, Alkoxycarbonyl, -NH-CO-NR₅R₆, -N(alk)-CO-NR₅R₆, -N(alk-Ar)-CO-NR₅R₆, -NH-CS-NR₅R₆, -N(alk) -CS-NR₅R₆, -NH-CO-R₅, -NH-CS-R₇, -NH-C(=NR₉)-NR₈R₆, -N(alk)-C(=NR₉)-NR₈R₆, -CO-NR₈R₆, -NH-SO₂-NR₈R₆, -N(alk)-SO₂-NR₈R₆, -NH-SO₂-CF₃, -NH-SO₂-alk, -NH-SO₂-Ar, -NR₈R₁₀, -S(O)ₘ-alk-Ar, -SO₂-NR₈R₆ oder 2-Oxo-1-imidazolidinyl, dessen 3-Stellung gegebenenfalls durch einen Alkylrest substituiert ist, oder 2-Oxo-1-perhydropyrimidinyl, dessen 3-Stellung gegebenenfalls durch einen Alkylrest substituiert ist, bedeuten,
- R₃ und R₄ gemeinsam mit dem Kohlenstoffatom, an das sie gebunden sind, einen 2- oder 3-Pyrrolidinring, einen 2- oder 4-Piperidinring, einen 2-Azacycloheptanring bilden, wobei diese Ringe gegebenenfalls an dem Stickstoff substituiert sind durch einen Rest Alkyl, -CHO, -COOR₁, -CG-alk-COOR₆, -CO-alk-NR₆R₁₂, -CO-alk-CONR₆R₈, -CO-COOR6, -CO-CH₂-O-CH₂-COOR₆, -CO-CH₂-S-CH₂-COOR₆, -CO-CH=CH-COOR_{6'} -CO-alk_{'} -CO-Ar", -CO-alk-Ar", -CO-NH-Ar", -CO-NH-alk-Ar", -CO-Het, -CO-alk-Het, -CO-NH-Het, -CO-NH-alk-Het, -CO-NH₂, -CO-NH-alk, -CO-N(alk)-alk', -CS-NH₂, -CS-NH-alk, -CS-NH-Ar", -CS-NH-Het, -alk-Het, alk-NR₆R₈, -alk-COOR₆, -alk-CO-NR₆R₈, -alk-Ar", -SO₂-alk, -SO₂-Ar oder -CO-cycloalkyl, dessen Cycloalkyl gegebenenfalls in 2-Stellung durch einen Carboxyrest substituiert ist,
- R₅ ein Wasserstoffatom oder einen Alkylrest (1-9C in gerader oder verzweigter Kette), -alk-COOR₈, -alk-Het, -alk-NR₆R₈, Phenylalkyl, dessen Phenylring gegebenenfalls durch einen oder mehrere Substituenten, ausgewählt unter den Halogenatomen und den Resten Alkyl, Alkoxy, Nitro, Amino, Hydroxy, -alk-NH₂, Carboxy, Alkoxycarbonyl, Cyano und -alk-COOR₈, substituiert ist, Phenyl, das gegebenenfalls substituiert ist durch einen oder mehrere Substituenten, ausgewählt unter den Halogenatomen und den Resten Alkyl, Alkoxy, Nitro, Amino, Hydroxy, -alk-NH₂, Carboxy, Alkoxycarbonyl, Cyano und -alk-COOR₈, oder -Het wiedergibt,
- R₆ für ein Wasserstoffatom oder einen Alkylrest steht,
- R₇ für einen Alkyl- oder Phenylrest steht,
- R₈ für ein Wasserstoffatom oder einen Alkylrest steht,
- R₉ für ein Wasserstoffatom oder einen Alkylrest steht,
- R₁₀ für einen Alkylrest, Het oder Alkoxycarbonyl steht,
- R₁₁ für einen Alkyl- oder Phenylalkylrest steht,
- R₁₂ für ein Wasserstoffatom oder einen Alkylrest steht,
- alk einen Alkylrest oder Alkylen bedeutet,
- alk' einen Alkylrest wiedergibt,
- m für 0, 1 oder 2 steht,
- Ar für einen Phenylrest steht,
- Ar" einer Phenylrest oder Phenyl, substituiert durch einen oder mehrere Substituenten, ausgewählt unter de Halogenatomen und den Resten Alkyl, Alkoxy, Nitro, Amino, Hydroxy, Cyano, -alk-NH₂, COOR₆ und -alk-COOR₆, bedeutet,
- Het einen gesättigten oder ungesättigten mono- oder polycyclischen Heterocyclus mit 1 bis 9 Kohlenstoffatomen und
einem oder mehreren Heteroatomen (O, S, N), der gegebenenfalls substituiert ist durch einen oder mehrere Alkyl-, Phenyl-, Carboxy- oder Phenylalkylreste, darstellt,
wobei, sofern nicht erwähnt, die Alkyl-, Alkylen- und Alkoxyreste und -teile 1 bis 6 Kohlenstoffatome enthalten und in gerader oder verzweigter Kette vorliegen und die Cycloalkylreste 3 bis 6 Kohlenstoffatomen enthalten,
die Enantiomeren undDiastereomeren der Verbindungen der Formel (I), bei denen R₃ und R₄ gemeinsam mit dem Kohlenstoffatom, an das sie gebunden sind, einen 2- oder 3-Pyrrolidin-, 2-Piperidin- oder 2-Azacycloheptanring bilden, wobei die cis- und trans-Isomeren der Verbindungen der Formel (I) einen Rest -CO-CH=CH-COOR₆ aufweisen,
und die Salze dieser Verbindungen.

2. Verbindungen der Formel (I) gemäß Anspruch 1, worin Het ausgewählt ist unter folgenden Ringen: Pyrrolyl, gegebenenfalls substituiert durch einen oder mehrere Alkyl-, Phenyl-, Carboxy- oder Phenylalkylreste; Pyridyl, gegebenenfalls substituiert durch einen oder mehrere Alkyl-, Phenyl-, Carboxy- oder Phenylalkylreste; Pyrimidinyl, gegebenenfalls substituiert durch einen oder mehrere Alkyl-, Phenyl-, Carboxy- oder Phenylalkylreste; Imidazolyl, gegebenenfalls substituiert durch einen oder mehrere Alkyl-, Phenyl-, Carboxy-oder Phenylalkylreste; Thiazolyl, gegebenenfalls substituiert durch einen oder mehrere Alkyl-, phenyl-, Carboxy- oder Phenylalkylreste; Oxazolinyl, gegebenenfalls substituiert durch einen oder mehrere Alkyl-, Phenyl-, Carboxy- oder Phenylalkylreste; Thiazolinyl, gegebenenfalls substituiert durch einen oder mehrere Alkyl-, Phenyl-, Carboxy- oder Phenylalkylreste; pyrazinyl, gegebenenfalls substituiert durch einen oder mehrere Alkyl-, Phenyl-, Carboxy- oder Phenylalkylreste; Tetrazolyl, gegebenenfalls substituiert durch einen oder mehrere Alkyl-, Phenyl-, Carboxy- oder Phenylalkylreste; oder Triazolyl, gegebenenfalls substituiert durch einen oder mehrere Alkyl-, Phenyl-, Carboxy- oder Phenylalkylreste, und deren Salze.

3. Verbindungen der Formel (I) gemäß Anspruch 1, worin R ein Wasserstoffatom oder einen Carboxy-, Alkoxycarbonyl-oder Carboxamidorest bedeutet, R₁ und R₂ Wasserstoffatome sind, R₃ und R₄ gemeinsam mit dem Kohlenstoffatom, an das sie gebunden sind, einen 2- oder 3-Pyrrolidin-, 2- oder 4-Piperidinring bilden, wobei diese Ringe gebenenfalls an dem Stickstoff durch einen Alkylrest oder einen Rest -CO-alk-COOR₆ substituiert sind, und deren Salze.

4. Die folgenden Verbindungen:
(10'RS)-Spiro[pyrrolidin-3,10'-5'H,10'H-imidazo[1,2-a ]-indeno[1,2-e]pyrazin]-4'-on,
(10'RS)-Spiro [piperidin-2,10'-5'H,10'H-imidazo[1,2-a]-indeno [1,2-e]pyrazin]-4'-on,
(10'RS)-Spiro[pyrrolidin-2,10'-5'H,10'H-imidazo[1,2-a]-indeno[1,2-e]pyrazin]-4'-on,
(10'RS)-1-Methyl-spiro[pyrrolidin-2,10'-5'H,10'H-imidazo[1,2-a]indeno[1,2-e]pyrazin]-4'-on,
(+)-1-Methyl-spiro[pyrrolidin-2,10'-5'H,10'H-imidazo[1,2-a]indeno[1,2-e]pyrazin]-4'-on,
(-)-1-Methyl-spiro[pyrrolidin-2,10'-5'H,10'H-imidazo[1,2-a]indeno[1,2-e]pyrazin]-4'-on,
(10'Rs)-4-Oxo-4-[4'-oxo-4',5'-dihydro-spiro[pyrrolidin-2,10'-10'H-imidazo[1,2-a]indeno[1,2-e]pyrazin-1-yl]]-buttersäure,
und deren Salze.

5. Verfahren zur Herstellung der Verbindungen der Formel (I) gemäß Anspruch 1, worin R₃ und R₄ mit dem Kohlenstoffatom, an das sie gebunden sind, einen 2-Pyrrolidin-, 2-Piperidin- oder 2-Azacycloheptanring bilden, dadurch gekennzeichnet, daß man ein Derivat der Formel 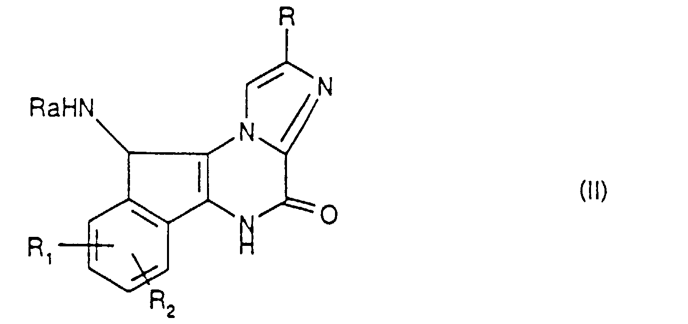 worin Ra eine Schutzgruppe für die Aminfunktion bedeutet, R, R₁ und R₂ die in Anspruch 1 angegebene Bedeutung besitzen, mit einem Derivat der Formel
Hal-(CH₂)ₚ-Hal' (III)
worin Hal und Hal', identisch oder verschieden, Halogenatome sind und p für 3, 4 oder 5 steht, umsetzt, hiernach das NH von der Schutzgruppe befreit, das Produkt isoliert und es gegebenenfalls in ein Salz überführt.

6. Verfahren zur Herstellung der Verbindungen der Formel (I) gemäß Anspruch 1, worin R₃ und R₄ mit den Kohlenstoffatom, an das sie gebunden sind, einen 3-Pyrrolidinring bilden, dadurch gekennzeichnet, daß man N-n-Butoxymethyl-N-trimethylsilylmethyl-benzylamin mit einem Derivat der Formel 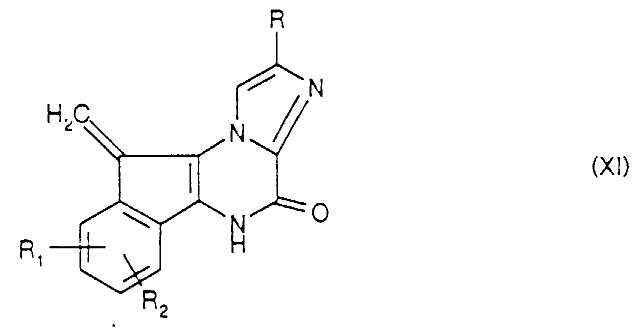 worin R, R₁ und R₂ die in Anspruch 1 angegebenen bedeutungen haben, umsetzt, hiernach das NH debenzyliert, das Produkt isoliert und es gegebenenfalls in ein Salz überführt.

7. Verfahren zur Herstellung der Verbindungen der Formel (I) gemäß Anspruch 1, worin R₃ und R₄ mit dem Stickstoffatom, an das sie gebunden sind, einen 4-Piperidinring bilden, dadurch gekennzeichnet, daß man N,N-Bis-(2-chlorethyl)-p-toluolsulfonamid mit einem Derivat der Formel 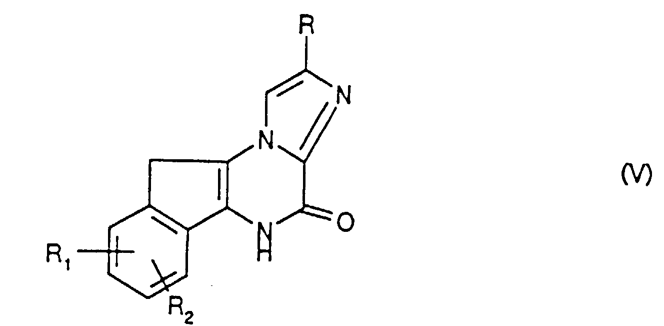 worin R, R₁ und R₂ die in Anspruch 1 angegebenen Bedeutungen besitzen, umsetzt, hiernach die Sulfonamidfunktion hydrolysiert, das Produkt isoliert und es gegebenenfalls in ein Salz überführt.

8. Verfahren zur Herstellung der Verbindungen der Formel (I) gemäß Anspruch 1, worin R₃ und R₄ mit dem Kohlenstoffatom, an das sie gebunden sind, einen 2- oder 3-Pyrrolidin-, 2- oder 4-Piperidin- oder 2-Azacycloheptanring, dessen Stickstoffatom durch einen 1C-Alkylrest substituiert ist, bilden, dadurch gekennzeichnet, daß man Formaldehyd und Ameisensäure mit einer entsprechenden Verbindung der Formel (I), worin R₃ und R₄ mit dem Kohlenstoffatom, an das sie gebunden sind, einen 2- oder 5-Pyrrolidin-, 2- oder 4-Piperidin- oder 2-Azacycloheptanring bilden, umsetzt, das Produkt isoliert und es gegebenenfalls in ein Salz überführt.

9. Verfahren zur Herstellung der Verbindungen der Formel (I) gemäß Anspruch 1, worin R₃ und R₄ mit de Kohlenstoffatom, an das sie gebunden sind, einen 2- oder 3-pyrrolidin-, oder 4-Piperidin- oder 2-Azacycloheptanring, deren Stickstoffatom durch einen (2-6C)-Alkylrest substituiert ist, bilden, dadurch gekennzeichnet, daß man eine Säure alk-COOH, worin alk einen 1-5C-alkylrest bedeutet, mit einer entsprechenden Verbindung der Formel (I), worin R₃ und R₄ mit dem Kohlenstoffatom, an das sie gebunden sind, einen 2- oder 3-Pyrrolidin-, 2- oder 4-Piperidin- oder 2-Azacycloheptanring bilden, in Gegenwart von Natriumborhydrid umsetzt, das Produkt isoliert und es gegebenenfalls in ein Salz überführt.

10. Verfahren zur Herstellung der Verbindungen der Formel (I) gemäß Anspruch 1, worin R₃ und R₄ mit den Kohlenstoffatom, an das sie gebunden sind, einen 2- oder 3-pyrrolidin-, 2- oder 4-Piperidin- oder 2-Azacycloheptanring bilden, deren Stickstoffatom substituiert ist durch einen Rest -CO-alk-NR₆R₁₂, worin R₆ und R₁₂ jeweils ein Wasserstoffatom bedeuten, dadurch gekennzeichnet, daß man eine entsprechende Verbindung der Formel (I), worin R₃ und R₄ mit dem Kohlenstoffatom, an das sie gebunden sind, einen 2- oder 3-pyrrolidin-, 2- oder 4-piperidin- oder 2-Azacycloheptanring bilden, mit einem Derivat der Formel 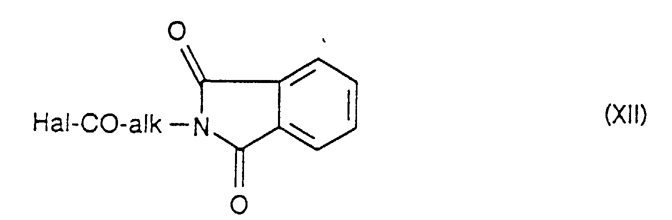 worin Hal für ein Halogenatom steht und alk einen Alkylrest bedeutet, umsetzt, hiernach das NH₂ von der Schutzgruppe befreit, das Produkt isoliert und es gegebenenfalls in ein Salz überführt.

11. Verfahren zur Herstellung der Verbindungen der Formel (I) gemäß Anspruch 1, worin R₃ und R₄ mit dem Kohlenstoffatom, an das sie gebunden sind, einen 2- oder 3-Pyrrolidin-, 2- oder 4-Piperidin- oder 2-Azacycloheptanring bilden, deren Stickstoffatom substituiert ist durch einen Rest (2-6C)-Alkyl, -COOR₁₁, -alk-Het, -alk-NR₆R₈, -alk-COOR₆, -alk-Ar", -CO-alk, -CC-alk-COOR₆, -CO-alk-NR₆R₁₂, -CO-COOR₆, -CO-cycloalkyl, dessen Cycloalkyl gegebenenfalls substituiert ist in 2-Stellung durch einen Rest Carboxy, -CO-Ar", -CO-alk-Ar", -CO-Het, -CO-alk-Het, -CO-Y(alk)alk', -SO₂-alk oder -SO₂-Ar, dadurch gekennzeichnet, daß man eine entsprechende Verbindung der Formel (I), worin und R₄ mit dem Kohlenstoffatom, an das sie gebunden sind, einen 2- oder 5-Pyrrolidin-, 2- oder 4-Piperidin- oder 2-Azacycloheptanring bilden, mit einem Halogenid Hal-Rc umsetzt, worin Hal ein Halogenatom bedeutet und Rc einen Rest (2-6C)-Alkyl, -COOR_{II}, -alk-Het, -alk-NR₆R₈, -alk-COOR₆, -alk-Ar", -CO-alk, -CO-alk-COOR₆, -CO-alk-NR₆R₁₂, -CO-COOR₆, -CO-(3-6C)-cycloalkyl darstellt, dessen Cycloalkyl gegebenenfalls in 2-Stellung substituiert ist durch einen Rest Carboxy, -CO-Ar", -CO-alk-Ar", -CO-Het, -CO-alk-Het, -CO-N(alk)-alk', -SO₂-alk oder -SO-Ar, wobei alk, alk' Het, R₆, R₈, R_{11'} R₁₂, Ar und Ar" die in Anspruch 1 angegebenen Bedeutungen besitzen, das Produkt isoliert und es gegebenenfalls in ein Salz überführt.

12. Verfahren zur Herstellung der Verbindungen der Formel (I) gemäß Anspruch 1, worin R₃ und R₄ mit dem Kohlenstoffatom, an das sie gebunden sind, einen 2- oder 3-Pyrrolidin-, 2- oder 4-Piperidin- oder 2-Azacycloheptanring bilden, deren Stickstoffatom durch einen Rest -CO-alk-CONR₆R₈ oder -alk-CO-NR₆R₈ substituiert ist, dadurch gekennzeichnet, daß man eine entsprechende Verbindung der Formel (I), worin R₃ und R₄ mit dem Kohlenstoffatom, an das sie gebunden sind, einen 2- oder 3-Pyrrolidin-, 2- oder 4-Piperidin- oder 2-Azacycloheptanring bilden, deren Stickstoffatom durch einen Rest -CO-alk-COOR₆ oder -alk-COOR₆ substituiert ist, mit einem Amin HNR₆R₈ worin R₆ und R₈ die in Anspruch 1 angegebenen Bedeutungen besitzen, umsetzt, das Produkt isoliert und es gegebenenfalls in ein Salz überführt.

13. Verfahren zur Herstellung der Verbindungen der Formel (I) gemäß Anspruch 1, worin R₃ und R₄ mit dem Kohlenstoffatom, an das sie gebunden sind, einen 2- oder 5-Pyrrolidin-, 2- oder 4-Piperidin- oder 2-Azacycloheptanring bilden, deren Stickstoffatom substituiert ist durch einen Rest -CHO, dadurch gekenzeichnet, daß man eine entsprechende Verbindung der Formel (I), in der R₃ und R₄ mit dem Kohlenstoffatom, an das sie gebunden sind, einen 2- oder 5-Pyrrolidin-, 2- oder 4-Piperidin- oder 2-Azacycloheptanring bilden, mit CH₃COOCHO umsetzt, das Produkt isoliert und es gegebenenfalls in ein Salz überführt.

14. Verfahren zur Herstellung der Verbindungen der Formel (I) gemäß Anspruch 1, worin R₃ und R₄ mit dem Kohlenstoffatom, an das sie gebunden sind, einen 2- oder 3-Pyrrolidin-, 2- oder 4-piperidin- oder 2-Azacycloheptan bilden, deren Stickstoffatom substituiert ist durch einen Rest -CO-Het, -CO-alk-COOR₆, -CO-COOR₆, -CO-alk-NR₆R₁₂, -CO-Ar", -CO-alk-Ar", -CO-alk-Het, -CO-alk- oder -CO-cycloalkyl, dessen Cycloalkylrest gegebenenfalls substituiert ist in der 2-Stellung durch einen Carboxyrest, dadurch gekennzeichnet, daß man eine entsprechende Verbindung der Formel (I), worin R₃ und R₄ mit dem Kohlenstoffatom, an das sie gebunden sind, einen 2- oder 3-pyrrolidin-, 2- oder 4-Piperidin- oder 2-Azacycloheptanring bilden, mit einem Derivat HO-Rd umsetzt, worin Rd einen Rest -CO-Het, -CO-alk-COOR₆, -CO-COOR₆, -CO-alk-NR₆R₁₂, -CO-Ar", -CO-alk-Ar", -CO-alk-Het, -CO-alk, -CO-(3-6C)-cycloalkyl bedeutet, deren Cycloalkyl gegebenenfalls in 2-Stellung durch einen Carboxyrest substituiert ist, wobei Het, alk, R₆, R₈, R₁₂, Het und Ar" die in Anspruch 1 angegebenen Bedeutungen besitzen, das Produkt isoliert und es gegebenenfalls in ein Salz überführt.

15. Verfahren zur Herstellung der Verbindungen der Formel (I) gemäß Anspruch 1, worin R₃ und R₄ mit dem Kohlenstoffatom, an das sie gebunden sind, einen 2- oder 5-Pyrrolidin-, 2- oder 4-Piperidin- oder 2-Azacycloheptanring bilden, deren Stickstoffatom substituiert ist durch einen Rest -CO-alk-COOR₆, worin alk 1 bis 3 Kohlenstoffatome in gerader Kette aufweist, -CO-CH₂-C(CH₃)₂-CH₂-COO₆, -CO-CH₂-CH₂-C(CH₃)₂-COOR₆, -CO-CH₂-C(CH₃)₂-COOR₆, -CO-CH₂-O-CH₂-COOR₆, -CO-CH₂-S-CH₂-COOR₆, -CO-CH=CH-COOR₆, -CO-(6C)-cycloalkyl, dessen Cycloalkyl substituiert ist in 2-Stellung durch einen Carboxyrest, -CO-Ar", worin Ar" einen Phenylrest, substituiert in 2-Stellung durch einen Carboxyrest, bedeutet, -CO-Het, worin Het einen 2- oder 4-Pyridylrest, substituiert in 3-Stellung durch einen Carboxyrest, oder 3-Pyridyl, substituiert in 4-Stellung durch einen Carboxyrest, bedeutet und R₆ ein Wasserstoffatom darstellt, dadurch gekennzeichnet, daß man ein Anhydrid der Formeln 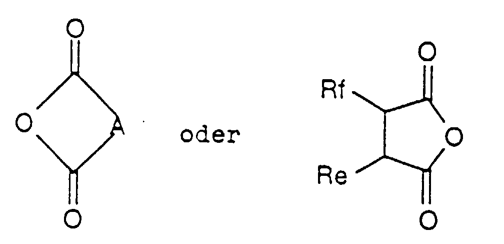 worin A einen Alkylrest (1-3C in gerader Kette), -CH₂-C(CH₃)₂-CH₂-, -CH₂-CH₂-C(CH₃)₂-, -CH₂-C(CH₃)₂-, -CH₂-O-CH₂-, -CH₂-S-CH₂, -CH=CH- bedeutet, Re und Rf gemeinsam mit den 2 Kolhenstoffatomen, ar die sie gebunden sind, einen Cycloalkyl(6C)-, Phenyl- oder Pyridylrest bedeuten, mit einer entsprechenden Verbindung der formel (I) umsetzt, worin R₃ und R₄ mit dem Kohlenstoffatom, an das sie gebunden sind, einen 2- oder 3-Pyrrolidin-, 2- oder 4-Piperidin- oder 2-Azacycloheptanring bilden, das Produkt isoliert und es gegebenenfalls in ein Salz überführt.

16. Verfahren zur Herstellung der Verbindungen der Formel (I) gemäß Anspruch 1, worin R₃ und R₄ gemeinsam mit dem Kohlenstoffatom, an das sie gebunden sind, einen 2- oder 3-Pyrrolidin-, 2- oder 4-Piperidin- oder 2-Azacycloheptanring bilden, deren Stickstoffatom substituiert ist durch einen Rest -CO-NH-alk-Ar", -CO-NH-Het, -CO-NH-alk-Het, -CO-NH-Ar", -CO-NH-alk, -CO-NH₂, -CSNH₂, -CS-NH-alk, -CS-NH-Ar" oder -CS-NH-Het, dadurch gekennzeichnet, daß man eine entsprechende Verbindung der Formel (I), worin R₃ und R₄ mit dem Kohlenstoffatom, an das sie gebunden sind, einen 2- oder 3-Pyrrolidin-, 2- oder 4-Piperidin- oder 2-Azacycloheptanring bilden, mit einen Derivat der Formel Rg=C=N-Rh umsetzt, worin Rg ein Sauerstoff- oder Schwefelatom bedeutet und Rh einen Rest Trimethylsilyl, Alkyl, Het, -alk-Ar", -alk-Het oder Ar"' wiedergibt, wobei Het, alk und Ar" die in Anspruch 1 angegebenen Bedeutungen besitzen, das Produkt isoliert und es gegebenenfalls in ein Salz überführt.

17. Verfahren zur Herstellung der Verbindungen der Formel (I) gemäß Anspruch 1, worin R einen Carboxyrest bedeutet, dadurch gekennzeichnet, daß man eine entsprechende Verbindung der Formel (I), worin R einen Alkoxycarbonylrest darstellg, hydrolysiert, das Produkt isoliert und es gegebenefalls in ein Salz überführt.

18. Arzneimittel, enthaltend als Wirkstoff zumindest eine Verbindung der Formel (I) gemäß Anspruch 1.

19. Arzneimittel, enthaltend als Wirkstoff zumindest eine Verbindung der Formel (I) gemäß einem der Anprüche 2 bis 4.

20. Arzneimittel gemäß einem der Ansprüche 18 und 19, verwendbar als Antagonist des AMPA-Rezeptors.

21. Arzneimittel gemäß einem der Ansprüche 18 und 19, verwendbar als Antagonisten des NMDA-Rezeptors.

## Claims

1. Compounds of formula: 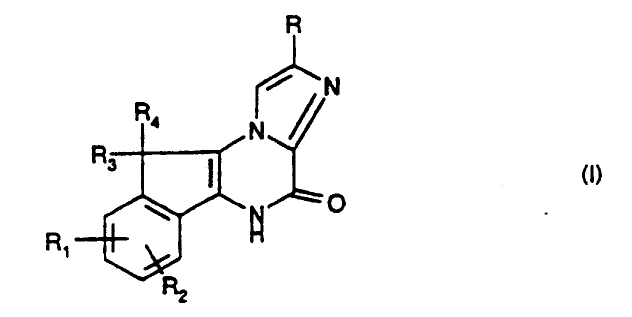 in which
- R represents a hydrogen atom or a carboxyl, alkoxycarbonyl or carboxamido radical,
- R₁ and R₂, which may be identical or different, represent hydrogen or halogen atoms or alkyl, alkoxy, amino, -N=CH-N(alk)alk', nitro, cyano, phenyl, imidazolyl, SO₃H, hydroxyl, polyfluoroalkoxy, carboxyl, alkoxycarbonyl, -NH-CO-NR₅R₆, -N (alk)-CO-NR₅R₆, -N(alk-Ar) -CO-NR₅R₆, -NH-CS-NR₅R₆, -N(alk)-CS-NR₅R₆, -NH-CO-R₅, -NH-CS-R₇, -NH-C (=NR₉) -NR₈R₆, -N(alk) -C (=NR₉) -NR₈R₆, -CO-NR₈R₆, -NH-SO₂-NR₈R₆, -N(alk)-SO₂-NR₈R₆, -NH-SO₂-CF₃, -NH-SO₂-alk, -NH-SO₂-Ar, -NR₈R₁₀, -S(O)ₘ-alk-Ar or -SO₂-NR₈R₆ radicals or a 2-oxo-1-imidazolidinyl radical in which the 3-position is optionally substituted with an alkyl radical, or a 2-oxoperhydro-l-pyrimidinyl radical in which the 3-position is optionally substituted with an alkyl radical,
- R₃ and R₄, together with the carbon atom to which they are attached, form a 2- or 3-pyrrolidine ring, a 2- or 4-piperidine ring or a 2-azacycloheptane ring, these rings optionally being substituted on the nitrogen with an alkyl, -CHO, -COOR₁₁, -CO-alk-COOR₆, -CO-alk-NR₆R₁₂, -CO-alk-CONR₆R₈, -CO-COOR₆, -CO-CH₂-O-CH₂-COOR₆, -CO-CH₂-S-CH₂-COOR₆, -CO-CH=CH-COOR₆, CO-alk, -CO-Ar", -CO-alk-Ar", -CO-NH-Ar", -CO-NH-alk-Ar", -CO-Het, -CO-alk-Het, -CO-NH-Het, -CO-NH-alk-Het, -CO-NH₂, -CO-NH-alk, -CO-N(alk)alk', -CS-NH₂, -CS-NH-alk, -CS-NH-Ar", -CS-NH-Het, -alk-Het, -alk-NR₆R₈, -alk-COOR₆, -alk-CO-NR₆R₈, -alk-Ar", -SO₂-alk or -SO₂-Ar radical or a -CO-cycloalkyl radical in which the cycloalkyl is optionally substituted in the 2-position with a carboxyl radical,
- R₅ represents a hydrogen atom or an alkyl (1-9C in a straight or branched chain), -alk-COOR₃, -alk-Het or -alk-NR₆R₈ radical, a phenylalkyl radical in which the phenyl ring is optionally substituted with one or more substituents chosen from halogen atoms and alkyl, alkoxy, nitro, amino, hydroxyl, -alk-NH₂, carboxyl, alkoxycarbonyl, cyano and -alk-COOR₈ radicals, a phenyl radical which is optionally substituted with one or more substituents chosen from halogen atoms and alkyl, alkoxy, nitro, amino, hydroxyl, -alk-NH2, carboxyl, alkoxycarbonyl, cyano and -alk-COOR₈ radicals, or a -Het radical,
- R₆ represents a hydrogen atom or an alkyl radical,
- R₇ represents an alkyl or phenyl radical,
- R₈ represents a hydrogen atom or an alkyl radical,
- R₉ represents a hydrogen atom or an alkyl radical,
- R₁₀ represents an alkyl, Het or alkoxycarbonyl radical,
- R₁₁ represents an alkyl or phenylalkyl radical,
- R₁₂ represents a hydrogen atom or an alkyl radical,
- alk represents an alkyl or alkylene radical,
- alk' represents an alkyl radical,
- m is equal to 0, 1 or 2,
- Ar represents a phenyl radical,
- Ar" is a phenyl radical or a phenyl radical substituted with one or more substituents chosen from halogen atoms and alkyl, alkoxy, nitro, amino, hydroxyl, cyano, -alk-NH₂, COOR₆ and -alk-COOR₆ radicals,
- Het represents a saturated or unsaturated mono- or polycyclic heterocycle containing 1 to 9 carbon atoms and one or more heteroatoms (O, S or N) which is optionally substituted with one or more alkyl, phenyl, carboxyl or phenylalkyl radicals,
it being understood that, except where otherwise mentioned, the alkyl, alkylene and alkoxy radicals and portions contain 1 to 6 carbon atoms and are in a straight or branched chain and the cycloalkyl radicals contain 3 to 6 carbon atoms,
the enantiomers and diastereoisomers of the compounds of formula (I) for which R₃ and R₄, together with the carbon atom to which they are attached, form a 2- or 3-pyrrolidine ring, a 2-piperidine ring or a 2-azacycloheptane ring, the cis and trans isomers of the compounds of formula (I) containing a radical -CO-CH=CH-COOR6
and the salts of these compounds.

2. Compounds of formula (I) according to claim 1 for which Het is chosen from a pyrrolyl ring optionally substituted with one or more alkyl, phenyl, carboxyl or phenylalkyl radicals, a pyridyl ring optionally substituted with one or more alkyl, phenyl, carboxyl or phenylalkyl radicals, a pyrimidinyl ring optionally substituted with one or more alkyl, phenyl, carboxyl or phenylalkyl radicals, an imidazolyl ring optionally substituted with one or more alkyl, phenyl, carboxyl or phenylalkyl radicals, a thiazolyl ring optionally substituted with one or more alkyl, phenyl, carboxyl or phenylalkyl radicals, an oxazolinyl ring optionally substituted with one or more alkyl, phenyl, carboxyl or phenylalkyl radicals, a thiazolinyl ring optionally substituted with one or more alkyl, phenyl, carboxyl or phenylalkyl radicals, a pyrazinyl ring optionally substituted with one or more alkyl, phenyl, carboxyl or phenylalkyl radicals, a tetrazolyl ring optionally substituted with one or more alkyl, phenyl, carboxyl or phenylalkyl radicals, or a triazolyl ring optionally substituted with one or more alkyl, phenyl, carboxyl or phenylalkyl radicals and salts thereof.

3. Compounds of formula (I) according to claim 1, for which R represents a hydrogen atom or a carboxyl, alkoxycarbonyl or carboxamido radical, R₁ and R₂ are hydrogen or halogen atoms, and R₃ and R₄, together with the carbon atom to which they are attached, form a 2- or 3-pyrrolidine ring or a 2- or 4-piperidine ring, these rings optionally being substituted on the nitrogen with an alkyl or -CO-alk-COOR₆ radical, and the salts thereof.

4. The following compounds:
- (10'RS)-spiro[pyrrolidine-3,10'-5'H,10'H-imidazo-[1,2-a]indeno[1,2-e]pyrazine]-4'-one,
- (10'RS)-spiro[piperidine-2,10'-5'H,10'H-imidazo-[1,2-a] indeno[1,2-e]pyrazine]-4'-one,
- (10'RS)-spiro[pyrrolidine-2,10'-5'H,10'H-imidazo-[1,2-a] indeno[1,2-e]pyrazine]-4'-one,
- (10'RS)-1-methylspiro[pyrrolidine-2,10'-5'H,10'H-imidazo[1,2-a]indeno[1,2-e]pyrazine]-4'-one,
- (+)-1-methylspiro[pyrrolidine-2,10'-5'H,10'H-imidazo[1,2-a] indeno[1,2-e]pyrazine]-4'-one,
- (-)-1-methylspiro[pyrrolidine-2,10'-5'H,10'H-imidazo[1,2-a]indeno[1,2-e]pyrazine]-4'-one,
- (10'RS)-4-oxo-4-{4'-oxo-4',5'-dihydrospiro-[pyrrolidine-2,10'-10'H-imidazo[1,2-a]indeno[1,2-e]pyrazin-1-yl]}butyric acid,
and the salts thereof.

5. Process for the preparation of the compounds of formula (I) according to claim 1, for which R₃ and R₄, together with the carbon atom to which they are attached, form a 2-pyrrolidine ring, a 2-piperidine ring or a 2-azacycloheptane ring, characterized in that a derivative of formula: 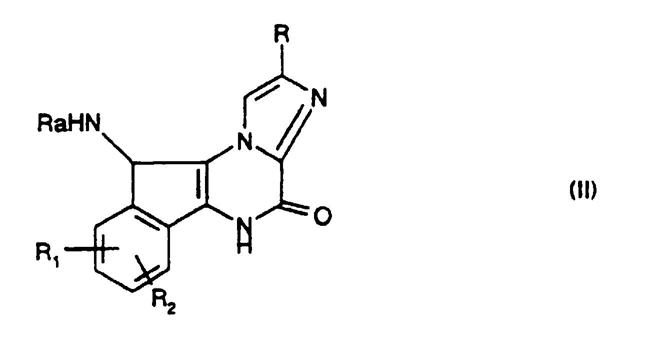 in which Ra represents a protecting group for the amine function and R, R₁ and R₂ have the same meanings as in claim 1, is reacted with a derivative of formula:
Hal-(CH₂)ₚ-Hal' (III)
in which Hal and Hal', which may be identical or different, are halogen atoms and p is equal to 3, 4 or 5, then the NH is deprotected and the product is isolated and optionally converted into a salt.

6. Process for the preparation of the compounds of formula (I) according to claim 1, for which R₃ and R₄, together with the carbon atom to which they are attached, form a 3-pyrrolidine ring, characterized in that N-n-butoxymethyl-N-trimethylsilylmethylbenzylamine is reacted with a derivative of formula: 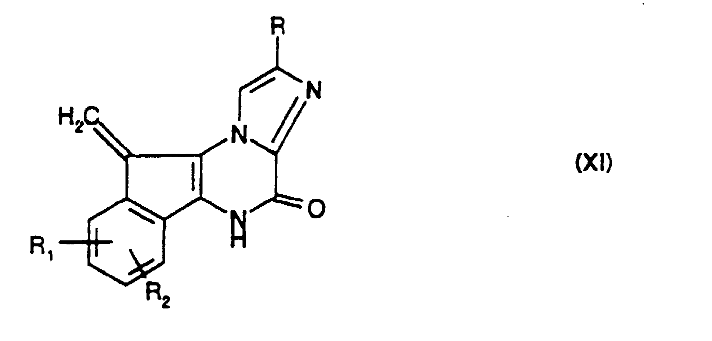 in which R, R₁ and R₂ have the same meanings as in claim 1, then the NH is debenzylated and the product is isolated and optionally converted into a salt.

7. Process for the preparation of the compounds of formula (I) according to claim 1, for which R₃ and R₄, together with the nitrogen atom to which they are attached, form a 4-piperidine ring, characterized in that N,N-bis (2-chloroethyl)-p-toluenesulphonamide is reacted with a derivative of formula: 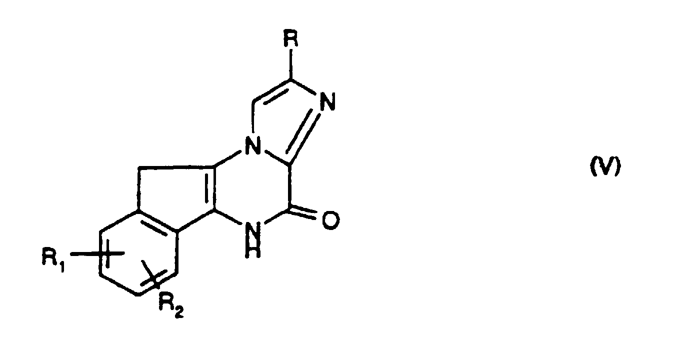 in which R, R₁ and R₂ have the same meanings as in claim 1, then the sulphonamide function is hydrolysed and the product is isolated and optionally converted into a salt.

8. Process for the preparation of the compounds of formula (I) according to claim 1, for which R₃ and R₄, together with the carbon atom to which they are attached, form a 2- or 3-pyrrolidine ring, a 2- or 4-piperidine ring or a 2-azacycloheptane ring in which the nitrogen atom is substituted with an alkyl 1C radical, characterized in that formaldehyde and formic acid are reacted with a corresponding compound of formula (I) for which R₃ and R₄, together with the carbon atom to which they are attached, form a 2- or 3-pyrrolidine ring, a 2- or 4-piperidine ring or a 2-azacycloheptane ring, and the product is isolated and optionally converted into a salt.

9. Process for the preparation of the compounds of formula (I) according to claim 1, for which R₃ and R₄, together with the carbon atom to which they are attached, form a 2- or 3-pyrrolidine ring, a 2- or 4-piperidine ring or a 2-azacycloheptane ring in which the nitrogen atom is substituted with an alkyl (2-6C) radical, characterized in that an acid alk-COOH in which alk represents an alkyl 1-5C radical is reacted with a corresponding compound of formula (I) for which R₃ and R₄, together with the carbon atom to which they are attached, form a 2- or 3-pyrrolidine ring, a 2- or 4-piperidine ring or a 2-azacycloheptane ring, in the presence of sodium borohydride, and the product is isolated and optionally converted into a salt.

10. Process for the preparation of the compounds of formula (I) according to claim 1, for which R₃ and R₄, together with the carbon atom to which they are attached, form a 2- or 3-pyrrolidine ring, a 2- or 4-piperidine ring or a 2-azacycloheptane ring in which the nitrogen atom is substituted with a radical -CO-alk-NR₆R₁₂ in which R₆ and R₁₂ each represent a hydrogen atom, characterized in that a corresponding compound of formula (I) for which R₃ and R₄, together with the carbon atom to which they are attached, form a 2- or 3-pyrrolidine ring, a 2- or 4-piperidine ring or a 2-azacycloheptane ring, is reacted with a derivative of formula: 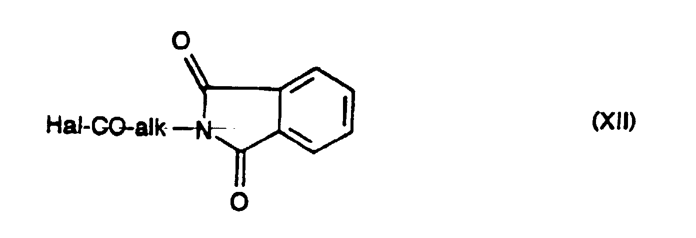 in which Hal represents a halogen atom and alk represents an alkyl radical, then the NH₂ is deprotected and the product is isolated and optionally converted into a salt.

11. Process for the preparation of the compounds of formula (I) according to claim 1, for which R₃ and R₄, together with the carbon atom to which they are attached, form a 2- or 3-pyrrolidine ring, a 2- or 4-piperidine ring or a 2-azacycloheptane ring in which the nitrogen atom is substituted with an alkyl (2-6C), -COOR₁₁, -alk-Het, -alk-NR₆R₈, -alk-COOR₆, -alk-Ar", -CO-alk, -CO-alk-COOR₆, -CO-alk-NR₆R₁₂ or -CO-COOR₆ radical, or a radical -CO-cycloalkyl in which the cycloalkyl is optionally substituted in the 2-position with a carboxyl, -CO-Ar", -CO-alk-Ar", -CO-Het, -CO-alk-Het,-CO-N(alk)alk', -SO₂-alk or -SO₂-Ar radical, characterized in that a corresponding compound of formula (I) for which R₃ and R₄, together with the carbon atom to which they are attached, form a 2- or 3-pyrrolidine ring, a 2- or 4-piperidine ring or a 2-azacycloheptane ring, is reacted with a halide Hal-Rc in which Hal represents a halogen atom and Rc represents an alkyl (2-6C), -COOR₁₁, -alk-Het, -alk-NR₆R₈, -alk-COOR₆, -alk-Ar", -CO-alk, -CO-alk-COOR₆, -CO-alk-NR₆R₁₂ or -CO-COOR₆ radical or a radical -CO-cycloalkyl (3-6C) in which the cycloalkyl is optionally substituted in the 2-position with a carboxyl, -CO-Ar", -CO-alk-Ar", -CO-Het, -CO-alk-Het, -CO-N(alk)alk', -SO₂-alk or -SO₂-Ar radical, alk, alk', Het, R₆, R₈, R₁₁, R₁₂, Ar and Ar" having the same meanings as in claim 1, and the product is isolated and optionally converted into a salt.

12. Process for the preparation of the compounds of formula (I) according to claim 1, for which R₃ and R₄, together with the carbon atom to which they are attached, form a 2- or 3-pyrrolidine ring, a 2- or 4-piperidine ring or a 2-azacycloheptane ring in which the nitrogen atom is substituted with a radical -CO-alk-CONR₆R₈ or -alk-CO-NR₆R₈, characterized in that a corresponding compound of formula (I) for which R₃ and R₄, together with the carbon atom to which they are attached, form a 2- or 3-pyrrolidine ring, a 2- or 4-piperidine ring or a 2-azacycloheptane ring in which the nitrogen atom is substituted with a radical -CO-alk-COOR6 or -alk-COOR6, is reacted with an amine HNR₆R₈ in which R₆ and R₈ have the same meanings as in claim 1, and the product is isolated and optionally converted into a salt.

13. Process for the preparation of the compounds of formula (I) according to claim 1, for which R₃ and R₄, together with the carbon atom to which they are attached, form a 2- or 3-pyrrolidine ring, a 2- or 4-piperidine ring or a 2-azacycloheptane ring in which the nitrogen atom is substituted with a -CHO radical, characterized in that a corresponding compound of formula (I) for which R₃ and R₄, together with the carbon atom to which they are attached, form a 2- or 3-pyrrolidine ring, a 2- or 4-piperidine ring or a 2-azacycloheptane ring, is reacted with CH₃COOCHO, and the product is isolated and optionally converted into a salt.

14. Process for the preparation of the compounds of formula (I) according to claim 1, for which R₃ and R₄, together with the carbon atom to which they are attached, form a 2- or 3-pyrrolidine ring, a 2- or 4-piperidine ring or a 2-azacycloheptane ring in which the nitrogen atom is substituted with a radical -CO-Het, -CO-alk-COOR₆, -CO-COOR₆, -CO-alk-NR₆R₁₂, -CO-Ar", -CO-alk-Ar", -CO-alk-Het or -CO-alk or a radical -CO-cycloalkyl in which the cycloalkyl is optionally substituted in the 2-position with a carboxyl radical, characterized in that a corresponding compound of formula (I) for which R₃ and R₄, together with the carbon atom to which they are attached, form a 2- or 3-pyrrolidine ring, a 2- or 4-piperidine ring or a 2-azacycloheptane ring, is reacted with a derivative HO-Rd in which Rd represents a radical -CO-Het, -CO-alk-COOR₆, -CO-COOR₆, -CO-alk-NR₆R₁₂, -CO-Ar", -CO-alk-Ar", -CO-alk-Het or -CO-alk, or a radical -CO-cycloalkyl(3-6C) in which the cycloalkyl is optionally substituted in the 2-position with a carboxyl radical, Het, alk, R₆, R₈, R₁₂, Het and Ar" having the same meanings as in claim 1, and the product is isolated and optionally converted into a salt.

15. Process for the preparation of the compounds of formula (I) according to claim 1, for which R₃ and R₄, together with the carbon atom to which they are attached, form a 2- or 3-pyrrolidine ring, a 2- or 4-piperidine ring or a 2-azacycloheptane ring in which the nitrogen atom is substituted with a radical -CO-alk-COOR₆ in which alk contains 1 to 3 carbon atoms in a straight chain, a radical -CO-CH₂-C(CH₃)₂-CH₂-COOR₆, -CO-CH₂-CH₂-C(CH₃)₂-COOR₆, -CO-CH₂-C(CH₃)₂-COOR₆, -CO-CH₂-O-CH₂-COOR₆, -CO-CH₂-S-CH₂-COOR₆ or -CO-CH=CH-COOR₆, a radical -CO-cycloalkyl (6C) in which the cycloalkyl is substituted in the 2-position with a carboxyl radical, a radical -CO-Ar" in which Ar" represents a phenyl radical substituted in the 2-position with a carboxyl radical, or a radical -CO-Het in which Het represents a 2- or 4-pyridyl radical substituted in the 3-position with a carboxyl radical or a 3-pyridyl radical substituted in the 4-position with a carboxyl radical and R₆ represents a hydrogen atom, characterized in that an anhydride of formulae: 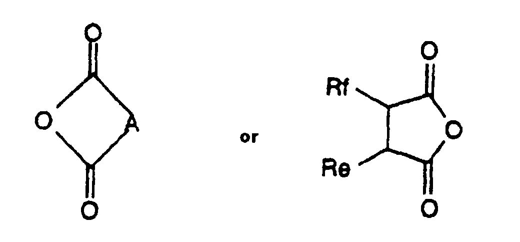 in which A represents an alkyl (1-3C in a straight chain), -CH₂-C(CH₃)₂-CH₂-, -CH₂-CH₂-C(CH₃)₂-, -CH₂-C(CH₃)₂-, -CH₂-O-CH₂-, -CH₂-S-CH₂ or -CH=CH- radical, Re and Rf form, together with the 2 carbon atoms to which they are attached, a cycloalkyl(6C), phenyl or pyridyl radical, is reacted with a corresponding compound of formula (I) for which R₃ and R₄, together with the carbon atom to which they are attached, form a 2- or 3-pyrrolidine ring, a 2- or 4-piperidine ring or a 2-azacycloheptane ring, and the product is isolated and optionally converted into a salt.

16. Process for the preparation of the compounds of formula (I) according to claim 1, for which R₃ and R₄, together with the carbon atom to which they are attached, form a 2- or 3-pyrrolidine ring, a 2- or 4-piperidine ring or a 2-azacycloheptane ring in which the nitrogen atom is substituted with a radical -CO-NH-alk-Ar", -CO-NH-Het, -CO-NH-alk-Het, -CO-NH-Ar", -CO-NH-alk, -CO-NH₂, -CSNH₂, -CS-NH-alk,-CS-NH-Ar" or -CS-NH-Het, characterized in that a corresponding compound of formula (I) for which R₃ and R₄, together with the carbon atom to which they are attached, form a 2- or 3-pyrrolidine ring, a 2- or 4-piperidine ring or a 2-azacycloheptane ring is reacted with a derivative of formula Rg=C=N-Rh in which Rg represents an oxygen or sulphur atom and Rh represents a trimethylsilyl, alkyl, Het, -alk-Ar", -alk-Het or Ar" radical, Het, alk and Ar" having the same meanings as in claim 1, and the product is isolated and optionally converted into a salt.

17. Process for the preparation of the compounds of formula (I) according to claim 1, for which R represents a carboxyl radical, characterized in that a corresponding compound of formula (I), for which R represents an alkoxycarbonyl radical, is hydrolysed, and the product is isolated and optionally converted into a salt.

18. Medicinal products containing at least one compound of formula (I) according to claim 1, as active principle.

19. Medicinal products containing at least one compound of formula (I) according to one of claims 2 to 4 as active principle.

20. Medicinal products according to either of claims 18 and 19, which are useful as AMPA-receptor antagonists.

21. Medicinal products according to either of claims 18 and 19, which are useful as NMDA-receptor antagonists.
